# EUROPEAN PATENT APPLICATION

(11) **EP 3 906 935 A1**
(43) Date of publication of application: **10.11.2021**
(21) Application number: 20305449.9
(22) Date of filing: 06.05.2020
(51) Int. Cl.: A61K 38/17, A61P 31/14, C07K 14/00, C12N 5/00

(54) **PEPTIDES FOR PREVENTING OR TREATING VIRAL INFECTIONS**

(71) Applicant: SORBONNE UNIVERSITE, 75006 Paris (FR); Centre National de la Recherche Scientifique, 75016 Paris (FR); Ecole Normale Supérieure, 75005 Paris (FR)
(72) Inventor: KAROYAN, Philippe, 91680 COURSON-MONTELOUP (FR); LEQUIN, Olivier, 75012 PARIS (FR)
(74) Representative: Gevers & Orès

(57) **Abstract**

The invention pertains to a new group of peptides that mimic an α-helix structure of the human Angiotensin Converting Enzyme 2 (hACE2) to bind the Spike protein (protein S) of viruses, in particular of coronaviruses. These peptides can be used in therapy, prophylaxis, diagnosis, medical imaging, drug screening and research.

## Description

The invention pertains to a new group of peptides that mimic an α-helix structure of the human Angiotensin Converting Enzyme 2 (hACE2) to bind the Spike protein (protein S) of viruses, in particular of coronaviruses. These peptides can be used in therapy, prophylaxis, diagnosis, medical imaging, drug screening and research.

The coronavirus disease 2019 (COVID-19), caused by the severe acute respiratory syndrome-coronavirus 2 (SARS-CoV-2) has emerged as a pandemic, claiming more than 117 000 deaths and around 1,8 million confirmed cases world-wide between December 2019 and April 2020. Since SARS-CoV-2 discovery and identification, the energy deployed by the scientific community has made it possible to generate an extraordinary amount of data, both in terms of quality and quantity. However, to date, clinically approved vaccines or drugs are lacking. Indeed, no specific drugs targeting this new virus are available, but many clinical trials have been engaged with non-specific treatments.

Many vaccine approaches are also currently under investigation at a pandemic speed. But referring to coronaviruses specialists, SARS-CoV-2 vaccine might be produced within 12 to 18 months. Indeed, vaccine development is a lengthy and expensive process. Attrition is high, and it typically takes multiple candidates and many years to produce an approved vaccine.

Regarding the development of specific drugs for SARS-CoV-2, such new drugs will likely not be small molecules. Indeed, apart from the time required for the screening and structure-activity relationship studies to identify a new compound and clinical development, small molecules drugs are associated to high attrition level because of their out-target toxicity detected during pharmacological studies. The multiple steps required to design such new drugs will be conducted with a high financial risk to developers and manufacturers and without knowing whether these drugs will be safe and effective, including very early manufacturing scale-up to commercial scale before establishment of clinical proof of concept.

Thus, there is a tremendous and urgent need for drugs that would be easier to design than small molecule drugs, associated with low or no toxicity and easy to produce on large scale.

The only class of drugs fitting for all these points are peptides. Peptides are widely recognized as promising therapeutic agents for the treatment of various conditions such as cancer, and metabolic, infectious or cardiovascular diseases. Across the United States, Europe, and Japan, to date more than 60 peptide drugs have reached the market and more than 150 are actually under clinical development. Special advantages that peptides show over other drugs include being highly versatile, target-specific, less toxic, and able to act on a wide variety of targets which are directly responsible for greater success rate than small molecules (approval rate of around 20% versus 10%).

In this context, the Inventors have developed an innovative approach using peptides to mimic a receptor targeted by the virus in order to block its infectivity, mainly in a preventive manner to stop the pandemic.

Beyond the characterization of the genome of SARS-CoV-2 and the identification of its probable origin by comparison with known SARS, MERS and Bat-coronavirus genomes, its infection mechanism has been deciphered highlighting that the virus cell-surface Spike protein (S protein) of SARS-CoV-2 is targeting human receptors. The human Angiotensin Converting Enzyme 2 (hACE2) and the cellular Transmembrane Protease Serine 2 (TMPRSS2) have been identified as major locks of the virus entry into human cells. In particular, the overexpression of hACE2 has been described as a possible risk factor for COVID-19 infection. Moreover, the virus-hACE2 interaction was already highlighted in the case of SARS-CoV. During viral infection, the trimeric S protein is cleaved into S 1 and S2 subunits and S 1 subunits are released in the transition to the post-fusion conformation. S1 contains the receptor binding domain (RBD), which directly binds to the peptidase domain (PD) of angiotensin-converting enzyme 2 (ACE2), whereas S2 is responsible for membrane fusion. When S 1 binds to the host receptor ACE2, another cleavage site on S2 is exposed and is cleaved by host proteases, a process that is critical for viral infection. Thus, blocking the virus-hACE2 interaction appears as a relevant therapeutic approach of coronavirus, and particularly for prophylaxis.

The peptides of the present invention have many advantages. They mimic hACE2, which is an extracellular target, easier to address that an intracellular one. Their bio-distribution can be restricted to the upper airways (oral cavity, ...) in a prophylactic approach and they will be degraded in the digestive tracks without any toxic residues. In case of blood stream access, they will have a short half-life and thus, they will not be toxic for humans. The intrinsic limitations for peptides generally include metabolic instability, which is the inability to withstand 600 proteases in the human body, and restriction to the parenteral route of administration. However, chemically synthesized peptides can overcome these challenges by incorporating additional entities, such as non-natural amino acids, to improve the metabolic stability, or other chemical entities, such as polyethylene glycols, to enhance membrane transportation. Nevertheless, this metabolic stability issue is not crucial in a prophylactic approach. Indeed, a peptide formulated for oral administration and release such as sublingual tablets for example, using only natural amino acids will be advantageously degraded in natural amino acids to avoid any risk of toxicity after metabolism of degradation. Moreover, to avoid any other toxicity issue, the peptides of the invention are designed *de novo* from a human protein, an endogenous target of the SARSCoV-2, but also a target of others coronavirus.

In an aspect, the invention thus relates to a peptide comprising or consisting in an amino acid sequence of SEQ ID NO: 1:
S-X-X-X-X-Q-X-X-T-F-X-D-K-X-X-H-E-X-E-[D/P/mA]-X-X-Y-Q-X-X-L (SEQ ID NO: 1),
wherein X is any amino acid, and
mA is a N-methyl-alanine,
or a pharmaceutically acceptable salt thereof.

The peptide according to the invention, which may be isolated, recombinant or synthetic, preferably synthetic, comprises or consists of an amino acid sequence.

This sequence defines a pharmacologically active peptide which mimics part of an α-helix structure from hACE2 that binds the Spike protein of viruses. This property can be readily verified by techniques known to those skilled in the art such as those described in the examples of the present application.

In the following description, the standard one letter amino acid code is used. The invention encompasses a peptide comprising or consisting of natural amino acids (20 gene-encoded amino acids in L- and/or D-configuration) linked via a peptide bond, as well as mimetics of such peptides wherein some amino acids and/or peptide bonds have been replaced by functional analogues. Such functional analogues include all known amino acids other than the 20 gene-encoded amino acids (or canonical amino acids). For example, a non-limitative list of non-coded amino acids (or non-canonical amino acids) is provided in Table 1A of US 2008/0234183. The invention also encompasses modified peptides derived from the above peptides by introduction of any modification into one or more amino acid residues, peptide bonds, N-and/or C-terminal ends of the peptide, as long as their property of binding to hACE2 is maintained in the modified peptide. These modifications which are introduced into the peptide by conventional methods known to those skilled in the art, include, in a non-limiting manner: the substitution of a natural amino acid with a non-proteinogenic amino acid (D amino acids or amino acid analogs); the modification of the peptide bond, in particular with a bond of the retro or retro-inverso type or a bond different from the peptide bond; the cyclization; the modification of the end(s) of the peptide, in particular a N-acetamido protection at the N-ter extremity and/or a C-carboxamide protection at C-ter extremity, and the addition of a chemical group to the side chain or the end(s) of the peptide, in particular for coupling an agent of interest to the peptide of the invention. These modifications may be used to label the peptide, and to increase its affinity for hACE2, its bioavailability, its stability, its half-life and/or to reduce its immunogenicity.

Pharmaceutically acceptable salts of the peptides disclosed herein are included in the present invention. For example, an acid salt of a compound containing an amine or other basic groups can be obtained by reacting the compound with a suitable organic or inorganic acid, resulting in pharmaceutically acceptable anionic salt forms. Examples of anionic salts include the acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate, triethiodide, and trifluoroacetate salts.

Salts of the compounds containing an acidic functional group can be prepared by reacting with a suitable base. Such a pharmaceutically acceptable salt can be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine.

In an embodiment, the peptide of the invention comprises or consists in an amino acid sequence of SEQ ID NO: 2:
S-X-X-E-X-Q-X-X-T-F-X-D-K-X-X-H-E-X-E-[D/P/mA]-X-X-Y-Q-X-X-L (SEQ ID NO: 2),
wherein X is any amino acid, and
m(A) is a N-methyl-alanine,
or a pharmaceutically acceptable salt thereof.

In an embodiment, the peptide of the invention comprises or consists in an amino acid sequence of SEQ ID NO: 3:
S-[T/L/A/V/I]-[I/L/A]-E-X-Q-X-X-T-F-X-D-K-X-X-H-E-X-E-[D/P/mA]-X-X-Y-Q-X-X-L (SEQ ID NO: 3),
wherein X is any amino acid, and
mA is a N-methyl-alanine,
or a pharmaceutically acceptable salt thereof.

In an embodiment, the peptide of the invention comprises or consists in an amino acid sequence of SEQ ID NO: 4:
S-T-I-E-X-Q-X-X-T-F-X-D-K-X-X-H-E-X-E-[D/P/mA]-X-X-Y-Q-X-X-L (SEQ ID NO: 4),
wherein X is any amino acid, and
mA is a N-methyl-alanine,
or a pharmaceutically acceptable salt thereof.

In an embodiment, the peptide of the invention comprises or consists in an amino acid sequence of SEQ ID NO: 5: 
S-[T/L/A/V/I]-[I/L/A]-E-E-Q-[A/L/Y/hY]-[K/A]-T-F-L-D-K-[F/A]-[N/L/M]-H-E-[A/L/]-E-
[D/P/mA]-[L/P]-[F/A/L]-Y-Q-[S/L/A]-[S/A]-L (SEQ ID NO: 5),
wherein hY is an homotyrosine, and
mA is a N-methyl-alanine,
or a pharmaceutically acceptable salt thereof.

In an embodiment, the peptide of the invention comprises or consists in an amino acid sequence of SEQ ID NO: 6: 
S-[T/L/A/V/I]-[I/L/A]-E-E-Q-[A/L/Y/hY]-[K/A]-T-F-L-D-K-[F/A]-M-H-E-[A/L/]-E-
[D/P/mA]-[L/P]-[F/A/L]-Y-Q-[S/L/A]-[S/A]-L (SEQ ID NO: 6),
wherein hY is an homotyrosine, and
mA is a N-methyl-alanine,
or a pharmaceutically acceptable salt thereof.

In an embodiment, the peptide of the invention peptide comprises or consists in an amino acid sequence of SEQ ID NO: 7:
S-X-X-X-X-Q-X-X-T-F-X-D-K-X-X-H-E-X-E-[D/P/mA]-X-X-Y-Q-X-X-L-X-X (SEQ ID NO: 7),
wherein X is any amino acid, and
mA is a N-methyl-alanine,
or a pharmaceutically acceptable salt thereof.

In an embodiment, the peptide of the invention peptide comprises or consists in an amino acid sequence of SEQ ID NO: 8:
S-X-X-E-X-Q-X-X-T-F-X-D-K-X-X-H-E-X-E-[D/P/mA]-X-X-Y-Q-X-X-L-X-X (SEQ ID NO: 8),
wherein X is any amino acid, and
mA is a N-methyl-alanine,
or a pharmaceutically acceptable salt thereof.

In an embodiment, the peptide of the invention comprises or consists in an amino acid sequence of SEQ ID NO: 9: 
S-[T/L/A/V/I]-[I/L/A]-E-X-Q-X-X-T-F-X-D-K-X-X-H-E-X-E-[D/PmA]-X-X-Y-Q-X-X-L-
X-X (SEQ ID NO: 9),
wherein X is any amino acid, and
mA is a N-methyl-alanine,
or a pharmaceutically acceptable salt thereof.

In an embodiment, the peptide of the invention comprises or consists in an amino acid sequence of SEQ ID NO: 10:
S-T-I-E-X-Q-X-X-T-F-X-D-K-X-X-H-E-X-E-[D/P/mA]-X-X-Y-Q-X-X-L-X-X (SEQ ID NO: 10),
wherein X is any amino acid, and
mA is a N-methyl-alanine,
or a pharmaceutically acceptable salt thereof.

In an embodiment, the peptide of the invention comprises or consists in an amino acid sequence of SEQ ID NO: 11: 
S-[T/L/A/V/I]-[I/L/A]-E-E-Q-[A/L/Y/hY]-[K/A]-T-F-L-D-K-[F/A]-[N/L/M]-H-E-[A/L/]-E-
[D/P/mA]-[L/P]-[F/A/L]-Y-Q-[S/L/A]-[S/A]-L-X-X (SEQ ID NO: 11),
wherein hY is an homotyrosine, and
mA is a N-methyl-alanine,
or a pharmaceutically acceptable salt thereof.

In an embodiment, the peptide of the invention comprises or consists in an amino acid sequence of SEQ ID NO: 12: 
S-[T/L/A/V/I]-[I/L/A]-E-E-Q-[A/L/Y/hY]-[K/A]-T-F-L-D-K-[F/A]-M-H-E-[A/L/]-E-
[D/P/mA]-[L/P]-[F/A/L]-Y-Q-[S/L/A]-[S/A]-L-X-X (SEQ ID NO: 12),
wherein hY is an homotyrosine, and
mA is a N-methyl-alanine,
or a pharmaceutically acceptable salt thereof.

In an embodiment, the peptide of the invention comprises or consists in an amino acid sequence of SEQ ID NO: 13: 
S-[T/L/A/V/I]-[I/L/A]-E-E-Q-[A/L/Y/hY]-[K/A]-T-F-L-D-K-[F/A]-[N/L/M]-H-E-[A/L/]-E-
[D/P/mA]-[L/P]-[F/A/L]-Y-Q-[S/L/A]-[S/A]-L-A-[S/L] (SEQ ID NO: 13),
wherein hY is an homotyrosine, and
mA is a N-methyl-alanine,
or a pharmaceutically acceptable salt thereof.

In an embodiment, the peptide of the invention comprises or consists in an amino acid sequence of SEQ ID NO: 14: 
S-[T/L/A/V/I]-[I/L/A]-E-E-Q-[A/L/Y/hY]-[K/A]-T-F-L-D-K-[F/A]-M-H-E-[A/L/]-E-
[D/P/mA]-[L/P]-[F/A/L]-Y-Q-[S/L/A]-[S/A]-L-A-[S/L] (SEQ ID NO: 14),
wherein hY is an homotyrosine, and
mA is a N-methyl-alanine,
or a pharmaceutically acceptable salt thereof.

A non-exhaustive list of peptides according to the invention is given in Table 1.

**Table 1. Non-exhaustive list of peptides according to the invention.**

| | |
|---|---|
| STIEEQAKTFLDKFNHEAEDLFYQSSL | SEQ ID NO: 15 |
| STIEEQAKTFLDKFLHEAEDLFYQSSL | SEQ ID NO: 16 |
| STIEEQAKTFLDKFMHEAEDLFYQSSL | SEQ ID NO: 17 |
| STIEEQLKTFLDKFLHEAEDLFYQSSL | SEQ ID NO: 18 |
| STIEEQLKTFLDKFNHEAEDLFYQSSL | SEQ ID NO: 19 |
| STIEEQLKTFLDKFMHEAEDLFYQSSL | SEQ ID NO: 20 |
| STIEEQLKTFLDKFAHEAEDLFYQSSL | SEQ ID NO: 21 |
| STIEEQLKTFLDKFLHELEDLFYQSSL | SEQ ID NO: 22 |
| STIEEQLKTFLDKFNHELEDLFYQSSL | SEQ ID NO: 23 |
| STIEEQLKTFLDKFMHELEDLFYQSSL | SEQ ID NO: 24 |
| SLIEEQLKTFLDKFLHELEDLFYQSSL | SEQ ID NO: 25 |
| SLIEEQLKTFLDKFNHELEDLFYQSSL | SEQ ID NO: 26 |
| SLIEEQLKTFLDKFMHELEDLFYQSSL | SEQ ID NO: 27 |
| STLEEQLKTFLDKFLHELEDLFYQSSL | SEQ ID NO: 28 |
| SLAEEQLKTFLDKFLHELEDLFYQSSL | SEQ ID NO: 29 |
| SALEEQLKTFLDKFLHELEDLFYQSSL | SEQ ID NO: 30 |
| SALEEQLKTFLDKFNHELEDLFYQSSL | SEQ ID NO: 31 |
| SALEEQLKTFLDKFMHELEDLFYQSSL | SEQ ID NO: 32 |
| SALEEQLKTFLDKFLHELEDLFYQASL | SEQ ID NO: 33 |
| SALEEQLKTFLDKFNHELEDLFYQASL | SEQ ID NO: 34 |
| SALEEQLKTFLDKFMHELEDLFYQASL | SEQ ID NO: 35 |
| SALEEQLKTFLDKFLHELEDLFYQSAL | SEQ ID NO: 36 |
| SALEEQLKTFLDKFNHELEDLFYQSAL | SEQ ID NO: 37 |
| SALEEQLKTFLDKFLHELEDLFYQAAL | SEQ ID NO: 38 |
| SALEEQLKTFLDKFNHELEDLFYQAAL | SEQ ID NO: 39 |
| SALAEQLKTFLDKFLHELEDLFYQASL | SEQ ID NO: 40 |
| SALEEQLATFLDKFLHELEDLFYQASL | SEQ ID NO: 41 |
| SALEEQLKTFLDKALHELEDLFYQASL | SEQ ID NO: 42 |
| SALEEQLKTFLDKFLHELEDLAYQASL | SEQ ID NO: 43 |
| SALEEQLKTFLDKFNHELEDLAYQASL | SEQ ID NO: 44 |
| SALEEQLKTFLDKFMHELEDLAYQASL | SEQ ID NO: 45 |
| SALEEQLKTFLDKFLHELEDLAYQSSL | SEQ ID NO: 46 |
| SALEEQLKTFLDKFLHELEDLAYQLSL | SEQ ID NO: 47 |
| SALEEQLKTFLDKFLHELEDLLYQASL | SEQ ID NO: 48 |
| SALEEQLKTFLDKFNHELEDLLYQASL | SEQ ID NO: 49 |
| SALEEQLKTFLDKFMHELEDLLYQASL | SEQ ID NO: 50 |
| SLLEEQLKTFLDKFLHELEDLAYQASL | SEQ ID NO: 51 |
| SALEEQLKTFLDKFLHELEDLLYQLSL | SEQ ID NO: 52 |
| SALEEQLKTFLDKFNHELEDLLYQLSL | SEQ ID NO: 53 |
| SALEEQLKTFLDKFMHELEDLLYQLSL | SEQ ID NO: 54 |
| SALEEQLKTFLDKFLHELEDLLYQLAL | SEQ ID NO: 55 |
| SALEEQLKTFLDKFNHELEDLLYQLAL | SEQ ID NO: 56 |
| SALEEQLKTFLDKFMHELEDLLYQLAL | SEQ ID NO: 57 |
| SAIEEQLKTFLDKFLHELEDLLYQLAL | SEQ ID NO: 58 |
| SAIEEQLKTFLDKFNHELEDLLYQLAL | SEQ ID NO: 59 |
| SAIEEQLKTFLDKFMHELEDLLYQLAL | SEQ ID NO: 60 |
| SVLEEQLKTFLDKFLHELEDLLYQLAL | SEQ ID NO: 61 |
| SILEEQLKTFLDKFLHELEDLLYQLAL | SEQ ID NO: 62 |
| SALEEQLKTFLDKFLHELEPLLYQLAL | SEQ ID NO: 63 |
| SALEEQLKTFLDKFLHELEDPLYQLAL | SEQ ID NO: 64 |
| SALEEQLKTFLDKFNHELEDPLYQLAL | SEQ ID NO: 65 |
| SALEEQLKTFLDKFMHELEDPLYQLAL | SEQ ID NO: 66 |
| STIEEQAKTFLDKFNHEAEDLFYQSSLAS | SEQ ID NO: 67 |
| SALEEQLKTFLDKFLHELEDLLYQLALAS | SEQ ID NO: 68 |
| SALEEQLKTFLDKFMHELEDLLYQLALAS | SEQ ID NO: 69 |
| SALEEQLKTFLDKFLHELEDLLYQLALAL | SEQ ID NO: 70 |
| SALEEQLKTFLDKFMHELEDLLYQLALAL | SEQ ID NO: 71 |
| SALEEQLKTFLDKFNHELEDLLYQLALAL | SEQ ID NO: 72 |
| SALEEQYKTFLDKFLHELEDLLYQLALAL | SEQ ID NO: 73 |
| SALEEQ (hY) KTFLDKFLHELEDLLYQLAL | SEQ ID NO: 74 |
| SALEEQ (hY) KTFLDKFNHELEDLLYQLAL | SEQ ID NO: 75 |
| SALEEQ (hY) KTFLDKFMHELEDLLYQLAL | SEQ ID NO: 76 |
| SALEEQ (hY) KTFLDKFLHELEDPLYQLALAL | SEQ ID NO: 77 |
| SALEEQ (hY) KTFLDKFNHELEDPLYQLAL | SEQ ID NO: 78 |
| SALEEQ (hY) KTFLDKFMHELEDPLYQLAL | SEQ ID NO: 79 |

In an embodiment, the peptide of the invention comprises or consists in an amino acid sequence selected from the group consisting of: SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79 and SEQ ID NO: 80.

In a preferred embodiment, the peptide of the invention comprises or consists in an amino acid sequence selected from the group consisting of: SEQ ID NO: 15, SEQ ID NO: 17, SEQ ID NO: 20, SEQ ID NO: 55, SEQ ID NO: 56, SEQ ID NO: 57, SEQ ID NO: 59, SEQ ID NO: 60, SEQ ID NO: 66, SEQ ID NO: 74, SEQ ID NO: 75, SEQ ID NO: 76 and SEQ ID NO: 79.

In an embodiment, the peptide of the invention does not comprise or consist in an amino acid sequence of SEQ ID NO: 15.

In an embodiment, the peptide of the invention does not comprise or consist in an amino acid sequence of SEQ ID NO: 67.

In an embodiment, the peptide is:
- a fully free peptide,
- a Nter-acetamido protected peptide,
- a Cter-carboxamide protected peptide, or
- a Nter-acetamido and Cter-carboxamide protected peptide.

Peptides consisting of SEQ ID NO: 80 to 145 are Cter-carboxamide protected. Peptide consisting of SEQ ID NO: 123 is Nter-acetamido and Cter-carboxamide protected.

In an embodiment, the peptide of the invention consists in an amino acid sequence selected from the group consisting of: SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128,, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, and SEQ ID NO: 145.

In a preferred embodiment, the peptide of the invention consists in an amino acid sequence selected from the group consisting of: SEQ ID NO: 80, SEQ ID NO: 82, SEQ ID NO: 85, SEQ ID NO: 120, SEQ ID NO: 121, SEQ ID NO: 122, SEQ ID NO: 125, SEQ ID NO: 126, SEQ ID NO: 132, SEQ ID NO:140, SEQ ID NO:141, SEQ ID NO: 142 and SEQ ID NO: 145.

In an embodiment, the peptide of the invention only contains canonical (or natural) amino acids.

In an embodiment, the peptide of the invention contains at least one non-canonical (or unnatural) amino acid.

In an embodiment, the size of the peptide of the invention exceeds 27 amino acids length.

In an embodiment, the size of the peptide of the invention is from 27 to 50 amino acids length.

In an embodiment, the size of the peptide of the invention is 27, 28, 29, 30, 31, 32, 33, 34, 35, 36, 37, 38, 39, 40, 41, 42, 43, 44, 45, 46, 47, 48, 49 or 50 amino acids length.

In an embodiment, the peptide of the invention has a helical content of at least 30%, preferably at least 40%, more preferably 50%.

In an embodiment, the peptide of the invention has an helical content of at least 30%, 35%, 40%, 45%, 50%, 55%, 60%, 65%, 70%, 75%, 80%, 85%, 90% or 95%, preferably of at least 50%.

The helical content can be determined using techniques known to those skilled in the art, such as those described in the examples of the present application. Preferably, the helical content is calculated using the Agadir program (Muñoz & Serrano, 1994, Nature Struct. Biol., 1:399-409).

In an embodiment, the peptide of the invention is not or low immunogenic.

The immunogenicity can be evaluated using techniques known to those skilled in the art, such as those described in the examples of the present application. Preferably, the immunogenicity is evaluated using the method of Kolaskar & Tongaonkar (FEBS Lett. 1990, 276(1-2): 172-174).

In an embodiment, the peptide of the invention is labeled (or tagged).

In an embodiment, the peptide of the invention is a chimeric peptide.

In such an embodiment, the peptide of the invention may comprise one or more other peptide moieties including some which can favor the purification, detection, immobilization of the peptide, and/or which increase its affinity for the protein S, its bioavailability, its production in expression systems and/or its stability. For example, such moieties may be selected from a labeling moiety (such as a fluorescent protein (GFP and its derivatives, BFP and YFP)), a reporter moiety (such as an enzyme tag (luciferase, alkaline phosphatase, glutathione-S-transferase (GST), β-galactosidase), a binding moiety such as an epitope tag (polyHis6, FLAG, HA, myc.), a DNA-binding domain, a poly-lysine tag for immobilization onto a support, and a targeting moiety for addressing the chimeric peptide to a specific tissue compartment.

In an embodiment, the peptide of the invention is labeled (or tagged) with an agent allowing its detection by diagnostic techniques or medical imaging.

In an embodiment, the peptide of the invention is a multimer, in particular a homomultimer.

As used herein, a homomultimer refers to a peptide according to the invention wherein the sequence corresponding to the α-helix is repeated at least two times.

In an embodiment, the peptide of the invention is a homodimer or a homotrimer, preferably a homotrimer. Indeed, the Inventors have previously shown that multimerization, in particular homotrimerization, can improve potency of therapeutic peptides (Denèfle et al., J. Med. Chem. 2019, 62:7656-7668).

All the embodiments and features given above for the peptide of the invention apply *mutatis mutandis* to the other aspects of the invention described below which involve said peptide.

In an aspect, the invention relates to the peptide as defined above for use as a medicament.

The present invention also relates to a method of prevention and/or treatment in a subject in need thereof comprising administering to said subject an effective amount of the peptide as defined above.

The present invention also relates to the use of the peptide as defined above in the manufacture of a medicament.

The peptide of the invention can be administered to the subject in conjunction with an acceptable pharmaceutical carrier as part of a pharmaceutical composition.

Formulation of a composition according to the invention can vary, for example in order to obtain a delayed effect or according to the route of administration selected.

In an embodiment, the composition comprising the peptide is in the form of a solution, a syrup, a tablet, a pill, an eye-wash, a powder, a spray, a capsule, a gum, a pastille, a lipstick or an emulsion.

Suitable pharmaceutical carriers may contain inert ingredients which do not interact with the peptide. Standard pharmaceutical formulation techniques can be employed, such as those described in Remington's Pharmaceutical Sciences, Mack Publishing Company, Easton, PA. Suitable pharmaceutical carriers for parenteral administration include, for example, sterile water, physiological saline, bacteriostatic saline, phosphate-buffered saline, Hank's solution, Ringer's-lactate and the like.

In an embodiment, a composition according to the invention contains a peptide as defined above in a concentration of at least 10 nM, preferably at least 100 nM, more preferably 1 mM.

In an embodiment, the subject is an animal, preferably a mammal, more preferably a human.

In an aspect, the invention relates to a peptide as defined above, for use in the prevention and/or the treatment of a viral infection, preferably a coronavirus infection, more preferably an infection by a coronavirus selected from the list consisting of SARS-CoV, MERS-CoV and SARS-CoV2.

The present invention also relates to a method of prevention and/or treatment of a viral infection, preferably a coronavirus infection, more preferably an infection by a coronavirus selected from the list consisting of SARS-CoV, MERS-CoV and SARS-CoV2, in a subject in need thereof comprising administering to said subject an effective amount of a peptide as defined above.

The present invention also relates to the use of a peptide as defined above in the manufacture of a medicament for the prevention and/or the treatment of a viral infection, preferably a coronavirus infection, more preferably an infection by a coronavirus selected from the list consisting of SARS-CoV, MERS-CoV and SARS-CoV2.

The peptides of the invention are indeed able to bind the viral protein Spike, in particular the Spike protein of coronaviruses, more particularly the Spike protein of SARS-CoV, MERS-CoV and/or SARS-CoV2. This interaction can be readily verified by techniques known to those skilled in the art such as those described in the examples of the present application.

In a prophylaxis approach against the infection of coronaviruses, the peptide of the invention is preferably used to coat the oral cavity, the nasal cavity and/or the eye surface. In such an embodiment, the peptide of the invention is preferably administered to a subject in the form of a solution, a syrup, a tablet (such as a sublingual tablet), an eye-wash, a powder, a spray (such as an oral or nasal spray), a capsule, a gum, a pastille or an emulsion.

In a preferred embodiment, the present invention relates to a peptide as defined above for use in the prevention of SARS-CoV2 infection, wherein said peptide is administered in the form of a solution, a syrup, a tablet (such as a sublingual tablet), an eye-wash, a powder, a spray (such as an oral or nasal spray), a capsule, a gum, a pastille a lipstick or an emulsion.

In an aspect, the invention relates to a diagnostic or imaging reagent comprising a peptide as defined above.

A reagent according to the invention is particularly adapted for immunoassays, such as ELISA tests, and can be used for the detection of viral particles in biological samples, such as saliva or epithelial cells.

In an aspect, the invention also relates to the use of a peptide as defined above as a diagnostic or imaging reagent to detect viruses, in particular coronaviruses, more particularly SARS-CoV, MERS-CoV or SARS-CoV2.

In an embodiment, the peptide is linked covalently to a fluorescent or radioactive agent.

Covalent coupling of a labeling agent, for example a fluorescent or radioactive agent, to the peptide may be achieved by incorporating the labeling agent at the N-or C-terminal end of the peptide during chemical synthesis of the peptide, or incorporating a reactive group in a recombinant or synthetic peptide, and then using the group to link the labeling agent covalently.

A subject of the present invention is also the use of a peptide as defined above, *in vitro* or *ex vivo,* for diagnosing an infection by viruses, in particular by coronaviruses, more particularly by SARS-CoV, MERS-CoV or SARS-CoV2.

Another subject of the present invention is a peptide as defined above for use, *in vivo,* for diagnosing an infection by viruses, in particular by coronaviruses, more particularly by SARS-CoV, MERS-CoV or SARS-CoV2.

A subject of the present invention is also the use of a peptide as defined above, as a research tool, in particular for studying the Spike protein-hACE2 interaction.

In another aspect, the invention relates to a polynucleotide encoding a peptide as defined above. The isolated, synthetic or recombinant polynucleotide may be DNA, cDNA, RNA or combination thereof, either single- and/or double-stranded. Preferably the polynucleotide comprises a coding sequence which is optimized for the host in which the peptide is expressed.

In an aspect, the invention relates to an expression vector comprising a polynucleotide encoding a peptide as defined above. Preferably, said vector is an expression vector capable of expressing said polynucleotide when transfected or transformed into a host cell, such as a mammalian, bacterial or fungal cell. The polynucleotide is inserted into the expression vector in proper orientation and correct reading frame for expression. Preferably, the polynucleotide is operably linked to at least one transcriptional regulatory sequence and, optionally to at least one translational regulatory sequence. Recombinant vectors include usual vectors used in genetic engineering and gene therapy including for example plasmids and viral vectors.

In an aspect, the invention relates to a host cell modified with a polynucleotide as defined above or an expression vector as defined above.

The polynucleotide, vector, cell of the invention are useful for the production of the peptide of the invention using well-known recombinant DNA techniques.

In an aspect, the invention relates to a pharmaceutical composition, comprising at least: (i) a peptide as defined above, a polynucleotide encoding said peptide, and/or an expression vector comprising said polynucleotide, and (ii) a pharmaceutically acceptable carrier.

A further aspect of the invention relates to a polynucleotide, a vector and/or a modified host cell of the invention for use as a medicament.

The invention also provides also a kit comprising : (a) a container that contains one or more of: a peptide, polynucleotide, recombinant vector, modified host cell, pharmaceutical composition, diagnostic or imaging reagent of the invention, in solution or in lyophilized form; (b) optionally, a second container containing a diluent or reconstituting solution for the lyophilized formulation; and (c) optionally instructions for the use of the solution(s) and/or the reconstitution and/or use of the lyophilized formulation(s).

The polynucleotide according to the invention is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

The following figures and examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Molecular modeling of hACE2 and cell-surface Spike protein of SARS-CoV-2 from 6m0j. The molecular modeling has been focused on the interacting fragments of hACE2 protein and SARS-CoV-2 S protein.
**Figure 2****.** Highlighting the surface interaction of the α-helix of hACE2 and cell-surface Spike protein of SARS-CoV-2.
**Figure 3****.** Highlights on the hACE2 α-helix interacting with the cell-surface Spike protein of SARS-CoV-2. The essential interacting residues of the helix are shown in grey and the residues not directly involved in the interaction are shown in black.
**Figure 4****.** Highlights on side chains interactions. **A.** The α-helices of hACE2 highlighting the Y₈₃. **B.** A₂₅/hY₂₅ mutation superimposed with the hACE2 full protein with Y83.
**Figure 5****.** Pymol visualization and superimposition for selected peptide mimics (H26/H27) with the α-helix in its native form. Peptides were modeled using XPLOR-NIH 2.52 (Schwieters et al., Progr. NMR Spectroscopy, 2006, 48:47-62) and AMBER 14 programs (Case *et al.,* AMBER 14, University of California, San Francisco). Structures were visualized using Pymol v0.99 (The PyMOL Molecular Graphics System, Version 2.0 Schrödinger, LLC).

### EXAMPLES

### MATERIALS & METHODS

### 1. General chemistry

### 1.1. Peptides syntheses

Peptides were produced by GENECUST or manually synthesized from Fmoc-protected amino acids utilizing standard solid phase peptide synthesis (SPPS) methods. Solid-phase peptide syntheses were performed in polypropylene Torviq syringes (10 or 20 mL) fitted with a polyethylene porous disk at the bottom and closed with an appropriate piston. Solvent and soluble reagents were removed through back and forth movements. The appropriate protected amino acids were sequentially coupled using PyOxim/Oxyma as coupling reagents. The peptides were cleaved from the chlorotrityl or rink amide resin with classical cleavage cocktail TFA/TIS/H2O (95:2.5:2.5). The crude products were purified using preparative scale HPLC. The final products were characterized by analytical LCMS and NMR. All tested compounds were TFA salts and were at least 95% pure. Detailed NMR studies were performed for the relevant peptides and assignment tables are provided in the Supporting Information.

### 1.2. Analytics

Two methods were conducted for LC-MS analysis.
*Method A:* Analytical HPLC was conducted on a X-Select CSH C18 XP column (30 mm × 4.6 mm i.d., 2.5 µm), eluting with 0.1% formic acid in water (solvent A) and 0.1% formic acid in acetonitrile (solvent B), using the following elution gradient: 0-3.2 min, 0-50% B; 3.2-4 min, 100% B. Flow rate was 1.8 mL/min at 40 °C. The mass spectra (MS) were recorded on a Waters ZQ mass spectrometer using electrospray positive ionization [ES+ to give (MH)+ molecular ions] or electrospray negative ionization [ES- to give (MH)- molecular ions] modes. The cone voltage was 20 V.
*Method B:* Analytical HPLC was conducted on a X-Select CSH C18 XP column (30 mm × 4.6 mm i.d., 2.5 µm), eluting with 0.1% formic acid in water (solvent A) and 0.1% formic acid in acetonitrile (solvent B), using the following elution gradient: 0-3.2 min, 5-100% B; 3.2-4 min, 100% B. Flow rate was 1.8 mL/min at 40 °C. The mass spectra (MS) were recorded on a Waters ZQ mass spectrometer using electrospray positive ionization [ES+ to give (MH)+ molecular ions] or electrospray negative ionization [ES- to give (MH)- molecular ions] modes. The cone voltage was 20 V.

### 1.3. Purification

Preparative scale purification of peptides was performed by reverse phase HPLC on a Waters system consisting of a quaternary gradient module (Water 2535) and a dual wavelength UV/visible absorbance detector (Waters 2489), piloted by Empower Pro 3 software using the following columns: preparative Macherey- Nagel column (Nucleodur HTec, C18, 250 mm × 16 mm i.d., 5 µm, 110 Å) and preparative Higgins analytical column (Proto 200, C18, 150 mm × 20 mm i.d., 5 µm, 200 Å) at a flow rate of 14 mL/min and 20 mL/min, respectively. Small-scale crudes (<30 mg) were purified using semipreparative Ace column (Ace 5, C18, 250 mm × 10 mm i.d., 5 µm, 300 Å) at a flow rate of 5 mL/min. Purification gradients were chosen to get a ramp of approximately 1% solution B per minute in the interest area, and UV detection was done at 220 and 280 nm. Peptide fractions from purification were analyzed by LC-MS (method A or B depending of retention time) or by analytical HPLC on a Dionex system consisting of an automated LC system (Ultimate 3000) equipped with an autosampler, a pump block composed of two ternary gradient pumps, and a dual wavelength detector, piloted by Chromeleon software. All LC-MS or HPLC analyses were performed on C18 columns. The pure fractions were gathered according to their purity and then freeze-dried using an Alpha 2/4 freeze-dryer from Bioblock Scientific to get the expected peptide as a white powder. Final peptide purity (>95%) of the corresponding pooled fractions was checked by LC-MS using method A.

### 2. Peptide stability studies

### 2.1. Degradation assays in human serum & mouse plasma

To a mixture of 250 µL of human serum (or mouse plasma) and 750 µL of RPMI 1640 were added 20 µL of the peptide DMSO stock solution at 10 mM. The mixture was incubated at 37°C. Aliquots of 100 µL were removed from the medium at different times, mixed with 100 µL of TCA solution (6%) and incubated at 4°C for at least 15 min to precipitate all the serum proteins. After centrifugation at 12,000 rpm for 2 min, 50 µL of the supernatant were transferred to an injection vial and analyzed by HPLC with a linear gradient of MeCN in water (5 to 95% + 0.1% TFA). The relative concentrations of the remaining soluble peptides were calculated by integration of the absorbance at 220 nm as a function of the retention time (peak area).

### 2.2. Stability under chymotrypsin and trypsin incubation

A 0.6 mL microcentrifuge tube was charged with 180 µL of phosphate buffer pH 7.4, 10 µL of enzyme (0.05 mg/mL stock solution in phosphate buffer pH 7.4), 10 µL of peptide (10 mM stock solution in DMSO). The resulting reaction mixture was capped and incubated at room temperature for 3 hours. 20 µL of the crude reaction was quenched by addition of 180 µL of 50% water: 50% acetonitrile and was subjected to LCMS analysis.

### 3. Structural analyses

### 3.1. CD spectroscopy

CD experiments were acquired on a Jasco J-815 CD spectropolarimeter with a Peltier temperature-controlled cell holder (30°C) over the wavelength range 190-270 nm. Peptide samples were prepared at a concentration of 50 µM in 10 mM sodium phosphate buffer, pH 7.4, using a quartz cell of 1 mm path length. Measurements were taken every 0.2 nm at a scan rate of 10 nm/min.

### 3.2. NMR spectroscopy

Lyophilized peptide was dissolved at 1 mM concentration in 550 µL of H2O/D2O (90:10 v/v). Sodium 4,4-dimethyl-4-silapentane-1-sulfonate-d6 (DSS, from Sigma Aldrich) was added at a final concentration of 0.11 mM for chemical shift calibration. NMR experiments were recorded on a Bruker Avance III 500 MHz spectrometer equipped with a TCI 1H/13C/15N cryoprobe with Z-axis gradient. NMR spectra were processed with TopSpin 3.2 software (Bruker) and analysed with NMRFAM-SPARKY program.43 1H, 13C, and 15N resonances were assigned using ID 1H WATERGATE, 2D 1H-1H TOCSY (DIPSI-2 isotropic scheme of 80 ms duration), 2D 1H-1H ROESY (300 ms mixing time), 2D 1H-13C HSQC, 2D 1H-15N HSQC, and 2D 1H-13C HMBC recorded at 25°C. 1H chemical shift was referenced against DSS 1H signal and 13C, 15N chemical shifts were referenced indirectly. The chemical shift deviations were calculated as the differences between observed 1H, 13C chemical shifts and random coil values.33 3JHN Hα coupling constants were measured on ID 1H WATERGATE experiments recorded at 5 or 25°C, or on ID rows extracted from 2D TOCSY acquired with high resolution.

### 4. Biological assays

### 4.1. Binding affinity measurements by biolayer interferometry

Fc-tagged 2019-nCoV RBD-SD1 was immobilized to an anti-human capture (AHC) sensortip (FortéBio) using an Octet RED96e (FortéBio). The sensortip was then dipped into 100 nM PKCOVID19-X to measure association before being dipped into a well containing only running buffer composed of 10 mM HEPES pH 7.5, 150 mM NaCl, 3 mM EDTA, 0.05% Tween 20 and 1 mg/mL bovine serum albumin to measure dissociation. Data were reference subtracted and fit to a 1:1 binding model using Octet Data Analysis Software v11.1 (FortéBio).

### 4.2. Prophylactic effect of hACE2 peptide mimics

Cells and virus preparation. Vero E6 (Vero C1008, clone E6 - CRL-1586; ATCC) cells were cultured in Dulbecco's Modified Eagle Medium (DMEM) supplemented with non-essential amino acids (NEAA), penicillin/streptomycin (P/S), Hepes buffer and 10% (v/v) Fetal bovine serum (FBS).

Aliquots (0.5 mL) of LBA from two symptomatic patients infected by SRAS-Cov2 (samples #xx and #yy) were mixed with an equal volume of DMEM without FBS and supplemented with double concentration of P/S, and added to 80% confluent Vero E6 cells monolayer seeded into a 25 cm2 tissue culture flask. After 1h adsorption at 37°C, 3 mL of DMEM supplemented with 2% FBS and Amphotericin B were added. Twenty-four hours post-infection (hpi) another 2 mL of DMEM supplemented with 2% FBS and Amphotericin B were added. Five days post-infection (dpi) cells and supernatant were collected, aliquoted and stored at -80°C (PI). For secondary (P2) virus stock, Vero E6 cells seeded into 25 cm2 tissue culture flasks were infected with 0.5 mL of P1 stored aliquot, and infected cells and supernatant were collected 48 hpi and stored at -80°C. Virus were quantified by RT-PCR assay, as described (Ref). Briefly, Viral RNA was purified from 140 mL of cell culture supernatant using the QIAamp Viral RNA Mini Kit (QIAGEN), following the manufacturer's instructions. Subsequently, the purified RNA was used to perform the synthesis of first-strand cDNA, using the SuperScriptTM First-Strand Synthesis System for RT-PCR (Thermo Fisher Scientific), following the manufacturer's instruction.

### 4.3. Experiments of peptide-neutralization.

Vero E6 cells (4 × 105 cells/mL) were seeded into 96 wells plates in DMEM, supplemented with 2% FBS and treated with different concentrations of peptides (from x to y), or 1 ug/mL of anti-ACE-2 mAb (ref), as control. After 30 min at room temperature, cells were infected with X or Y moi of SRAS-Cov2 from the two different samples. All conditions were tested in quadruplicate.

Cell supernatants were collected at 48h and 72 post-infection for real-time PCR (RT PCR) analysis and Elisa using SARS-CoV-2 (2019-nCoV) Nucleoprotein / NP ELISA Kit from Sino biological. Furthermore, infection of Vero cells will be tested by flow cytometry using SARS-CoV / SARS-CoV-2 (COVID-19) Spike antibody [1A9] from Genetex by flow cytometry.

### EXAMPLE I. Modelling and analysis of the interaction between hACE2and the Spike protein of SARS-CoV-2

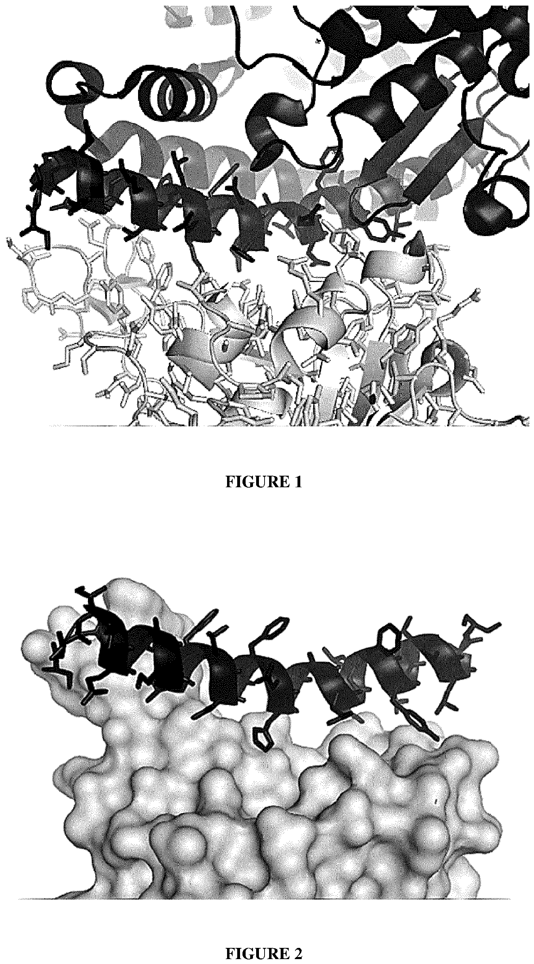

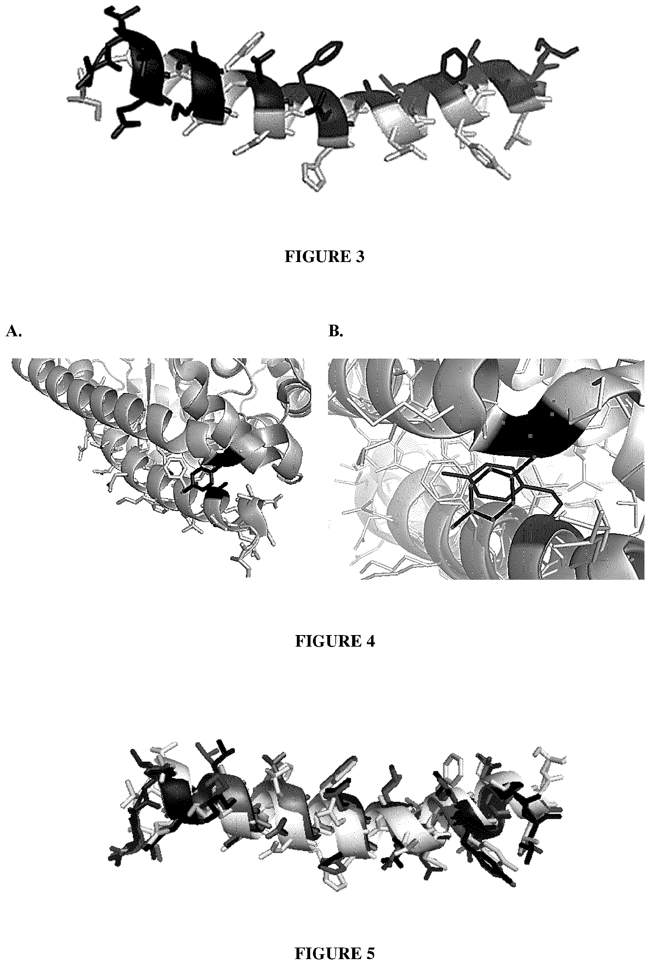

In order to highlight the contacts between hACE2 and cell-surface Spike protein (protein S) of SARS-CoV-2, the molecular modeling of the 6m0j file from PDB was performed (Figure 1). This file was chosen among the four available PDB structures to date. The molecular modeling has been focused on the interacting fragments of hACE2 protein and SARS-CoV-2 S protein. This contact occurs thanks to an α-helix of hACE2 on the surface of protein S of SARS-CoV-2 (Figure 2). This α-helix interacting with the protein S of SARS-CoV-2 is composed of 27 residues (hACE₁₉₋₄₅) as shown in SEQ ID NO: 15:
S₁₉T₂₀I₂₁E₂₂E₂₃Q₂₄A₂₅K₂₆T₂₇F₂₈L₂₉D₃₀K₃₁F₃₂N₃₃H₃₄E₃₅A₃₆E₃₇D₃₈L₃₉F₄₀Y₄₁Q₄₂S₄₃S₄₄L₄₅ (SEQ ID NO: 15),
wherein 13 crucial residues interact with the protein S (underlined) and 14 residues do not interact with the protein S but are possibly important for overall structure stabilization of the α-helix in the context of the whole hACE2 protein (Figure 3).

All the dihedral coordinates of each residues were calculated in agreement with the canonical values of dihedral angle of an α-helix but highlighting a kink around D₃₈ and L39. This observation might be relevant for the design of a peptide mimic of this peculiar helix, considering that D₃₈ side chain is crucial for the interaction whereas the one of F₄₀ is not.

The binding occurs throughout a series of hydrophilic interactions and few hydrophobic contacts (Figure 4). However, there are several noticeable contacts: T₂₇ interacts with a shallow hydrophobic pocket defined by A₄₇₅, Y₄₇₃ and Y₄₈₉; in the meantime, the carbon chain of K₃₁ nicely extends in a tiny groove defined by Y₄₈₉ and F₄₅₆ to finally hydrogen bonds with Q₄₉₃. F₂₈ and H₃₄ are both in weak interaction with the surface above F₄₈₆/Y₄₈₉ and L₄₅₅ respectively. There is along the helix a series of five main strong H bonds strengthening the interactions with the helix and the Spike surface, respectively Q₂₄ and N₄₈₇, K₃₁ and E₃₅ with both clipping Q₄₉₃, E₃₇ and Y₅₀₅, D₃₈ and Y₄₄₉, Y₄₁ and T₅₀₀ and finally head to head Q₄₂ and Q₄₉₈. H₃₄ also makes an H-bond with Y₄₅₃, and Y₄₁ with T₅₀₀ and N₅₀₁. Of note, out of the α-helix, Y₈₃ of hACE2 and F₄₈₆ of the protein S are in close proximity together with F₂₈. This interaction might stabilize the overall structure of the complex. Thus, it might be of importance to re-introduce the side chain of the Y₈₃ in the sequence of the α-helix to mimic this α-helix. For that purpose, A₂₅, which is not involved in any interaction, might be mutated to Y₂₅ or preferably to homoY₂₅. Indeed, MM calculation highlighted a better overlapping of aromatic ring with homoY instead of Y.

### EXAMPLE II. Design and characterization of α-helix peptide mimics

In the sequence of the α-helix, 13 residues (S₁₉, Q₂₄, T₂₇, F₂₈, D₃₀, K₃₁, H₃₄, E₃₅, E₃₇, D₃₈, Y₄₁, Q₄₂, L₄₅) appeared to be crucial for the interaction with protein S of SARS-Cov-2 and 14 residues (T₂₀, I₂₁, E₂₂, E₂₃, A₂₅, K₂₆, L₂₉, F₃₂, N₃₃, A₃₆, L₃₉, F₄₀, S₄₃, S₄₄) appeared to be non-essential for the interaction but may be important for helical sequence folding in the protein context. The following experiments were performed with the aim of designing a peptide with high helical folding propensity and able to retain most of the binding properties of hACE2 to the protein S of SARS-CoV-2 using mainly natural approaches and avoiding complex tools known to stabilize α-helix. Stabilizing the α-helical structure of medium size peptide sequences using only natural amino acids seemed to be achievable. For example, a peptide of eighteen residues with N- and C-protected extremities was previously built (Ac-EAEKAAKEAEKAAKEAEK-NH₂, SEQ ID NO: 146) (Zhou et al., Biochemistry 1993, 32:24, 6190-6197). This peptide was characterized by CD and NMR spectroscopies showing a content of 65% of amphipathic helical structure in solution.

The synthesis of the peptides of the invention was primarily directed by the will to provide a simple drug easy to produce quickly on a large scale, without technical constraints requiring sometimes laborious development. In this respect, the use of mostly natural amino acids was preferred since it can facilitate the essential stages of the development of therapeutic tools, particularly for pharmacokinetics, pre-clinical and clinical toxicity aspects. However, it is assumed that the design of the α-helix peptide mimics might be optimized regarding the binding affinity in term of entropy loss.

The sequence to mimic was analyzed in terms of helical folding propensities for each amino acid referring to Creighton reported values relative to the frequency of residues in secondary structures (Table 2):

**Table 2. Helical folding propensity per residue.**

| **Position in SEQ ID NO: 15** | **Amino acids** | **α-helix** | **Comments and possible substitutions to investigate** |
|---|---|---|---|
| 1 | S: Serine | 0,82 | Low frequency but crucial for capping box/ No substitution considered |
| 2 | T: Threonine | 0,82 | Low frequency / Not crucial/ T/A and T/L to investigate |
| 3 | I: Isoleucine | 0,92 | Low frequency / Not crucial/ I/A and I/L to investigate |
| 4 | E: Glutamate | 1,44 | High frequency / capping box/ Substitution not suitable |
| 5 | E: Glutamate | 1,44 | High frequency / Substitution not suitable |
| 6 | Q: Glutamine | 1,27 | High frequency / crucial / Substitution not suitable |
| 7 | A: Alanine | 1,29 | High frequency / Not crucial / A/L Substitution possible to increase helical content |
| 8 | K: Lysine | 1,23 | High frequency / Not crucial / K/L Substitution possible to increase helical content but at the expense of polarity and solubility |
| 9 | T: Threonine | 0,82 | Low frequency / Crucial interactions / Substitution not suitable |
| 10 | F: Phenylalanine | 1,07 | Average frequency / Crucial interactions / Substitution not suitable |
| 11 | L: Leucine | 1,30 | High frequency / Not crucial / Substitution not required |
| 12 | D: Aspartate | 1,04 | Average frequency / Crucial interactions / Substitution not suitable |
| 13 | K: Lysine | 1,23 | High frequency / Crucial interaction / Substitution not suitable |
| 14 | F: Phenylalanine | 1,07 | Average frequency / Not crucial / F/L and F/A substitutions to consider to increase helical content |
| 15 | N: Asparagine | 0,90 | Low frequency and known to destabilize helical content / N/A and N/L substitutions to investigate |
| 16 | H: Histidine | 1,22 | Average frequency / Crucial interactions / Substitution not suitable |
| 17 | E: Glutamate | 1,44 | High frequency / Crucial interaction / Substitution not suitable |
| 18 | A : Alanine | 1,29 | High frequency / Not crucial / A/L Substitution possible to increase helical content |
| 19 | E: Glutamate | 1,44 | High frequency / Involved in crucial interactions / Substitution not suitable |
| 20 | D: Aspartate | 1,04 | Average frequency / Involved in crucial interactions / Substitution not suitable |
| 21 | L: Leucine | 1,30 | High frequency / Not crucial / Substitution not required |
| 22 | F: Phenylalanine | 1,07 | Average frequency / Not crucial / F/L and F/A substitutions to consider to increase helical content |
| 23 | Y: Tyrosine | 0,72 | Low frequency / Crucial interactions / Substitution not suitable |
| 24 | Q: Glutamine | 1,27 | High frequency / Crucial interaction / Substitution not suitable |
| 25 | S: Serine | 0,82 | Low frequency / Not crucial /S/A and S/L substitutions to be considered |
| 26 | S: Serine | 0,82 | Low frequency / Not crucial /S/A and S/L substitutions to be considered |
| 27 | L: Leucine | 1,30 | High frequency / Crucial interaction / Substitution not suitable |
| X | M: Methionine | 1,47 | Highest frequency / Not present in the native helical helix but useful amino acid for substitution of not crucial residues in order to increase the helical content |

It was noted that the helical structure to mimic involves a favorable sequence in the N-terminus side around a "capping box", *i.e.* SX₁X₂E (SEQ ID NO: 147). This feature might be of crucial importance, especially for a peptide extracted from the hACE2 full protein context. This capping box involves a reciprocal backbone-side-chain hydrogen-bonding interaction between the oxygen atom of Serine side chain hydroxyl function and the Glutamate NH involved in the backbone, favoring thus the helix initiation. The X₁ and X₂ residues of the capping box of hACE2 correspond respectively to T and I. Since both residues are not involved in crucial interaction, increasing hydrophobicity in this position might enhance the helical propensity. The mutations of both T and I by residues favoring helical content will thus be considered.

In order to optimize the design of the α-helix peptide mimics, an iterative process was then set up using the Agadir program (http://agadir.crg.es), an algorithm developed to predict the helical content of peptides. The accuracy of this program was firstly evaluated by calculating the helical content of the 18 residues peptide of SEQ ID NO: 146 developed by Zhou *et al.,* at physiological pH7 and 278°K. Results are shown in Table 3.

**Table 3. Agadir calculation of the helical content of the 18 residues peptide developed by Zhou et al..**

| | | | | |
|---|---|---|---|---|
| Agadir Sequence(s) | EAEKAAKEAEKAAKEAEK (SEQ ID NO : 146) | | | |
| C-term | Amidated or free | | | |
| N-term | Acetylated or free | | | |
| pH | 7 | | | |
| Temperature | 278 | | | |
| Ionic Strength | 0.1 | | | |
| Results | 65,48% N-acetamide and C-carboxamide | 46,39% N-acetamide | 31,74% C-carboxamide | 15,42% Free peptide |

The theoretical calculation (65,48%) was in perfect agreement with the experimental data reported by Zhou *et al.* (65%), validating the accuracy of the Agadir algorithm for the design of peptide mimics of the α-helix of hACE2 protein. Of note, the calculation for the free N- and C-termini peptide led to a value of 15,42% of helical content, highlighting the potential crucial influence of N-capping (46,39% for Ac-N-capping and free C-termini while 31,74% for free N- and C-carboxamide termini) to preserve the helix macrodipole for this peculiar sequence of 18 residues.

Similar calculations performed for the 27mers of the hACE2 helix (SEQ ID NO: 15, hereafter named "Peptide **H1"**) led to poor helical content, whatever the N-and/or C-protection. Results are shown in Table 4.

**Table 4. Agadir calculation of the helical content of the 27mers residues hACE2 derived peptide.**

| | | | | |
|---|---|---|---|---|
| Agadir Sequence(s) | STIEEQAKTFLDKFNHEAEDLFYQSSL, peptide **H1** (SEQ ID NO : 15) | | | |
| C-term | Amidated or free | | | |
| N-term | Acetylated or free | | | |
| pH | 7 | | | |
| Temperature | 278 | | | |
| Ionic Strength | 0.1 | | | |
| Results | 3,34% N-acetamide and C-carboxamide | 3,42% N-acetamide | 5,51% C-carboxamide | 5,68% Free |

Regarding this sequence, the N-terminus amidation appeared to be deleterious. The information of Table 4 was used to initiate, step by step, the improvement of the helical folding propensity of the hACE2 derived 27mers peptide around the capping box and crucial residues. For each modification tested, the helical content was calculated for the fully free peptide (N-terminus: - NH3⁺ and C-terminus: -COO⁻), for the N-acetamido and C-carboxamide protected peptide and for respectively the N-terminus protected as acetamido or C-terminus protected as C-carboxamide. Results are summarized in Table 5.

**Table 5. Agadir calculation of the helical content of the 27mers peptide mimics. The calculation was realized for peptides fully protected or deprotected at the N-and C-termini, together with partial N- or C-protection (Data not shown). Only the values for the C-carboxamide peptides is here reported, except for peptide H22. *: I refers to a peptide designed for lowering calculated immunogenicity, H refers to a peptide designed for increasing helical content. **: Helical content % for C-carboxamide protected peptides. ***: AD means antigenic determinant, AAP means average antigenic propensity. §: a scramble peptide from peptide H1 was designed as a negative control for the binding and infectivity studies using a srambler tool. §§: a control peptide without the capping box was used.**

| Peptide name* | Peptide sequence | Helical content % ** | Immunogenicity (AD/AAP) *** | SEQ ID NO |
|---|---|---|---|---|
| **H1** | STIEEQAKTFLDKFNHEAEDLFYQSSL-NH₂ | 5,51 | (0/1,0083) | 80 |
| **H2** | STIEEQAKTFLDKFLHEAEDLFYQSSL-NH₂ | 8,33 | (1/1,0259) | 81 |
| **I1** | STIEEQAKTFLDKFMHEAEDLFYQSSL-NH₂ | 7,32 | (0/1,0102) | 82 |
| **H3** | STIEEQLKTFLDKFLHEAEDLFYQSSL-NH₂ | 21.02 | (1/1,0328) | 83 |
| **I3** | STIEEQLKTFLDKFNHEAEDLFYQSSL-NH₂ | 13,96 | (0/1,0152) | 84 |
| **H3/I3/N₃₃ /M₃₃** | STIEEQLKTFLDKFMHEAEDLFYQSSL-NH₂ | 18,62 | (0/1,0171) | 85 |
| **H3/I3** | STIEEQLKTFLDKFAHEAEDLFYQSSL-NH₂ | 18,18 | (2/1,0259) | 86 |
| **H4** | STIEEQLKTFLDKFLHELEDLFYQSSL-NH₂ | 32.54 | (1/1,0397) | 87 |
| **I4** | STIEEQLKTFLDKFNHELEDLFYQSSL-NH₂ | 14,97 | (0/1,0221) | 88 |
| **H4/I4/N₃₃ /M₃₃** | STIEEQLKTFLDKFMHELEDLFYQSSL-NH₂ | 25,88 | (0/1,0240) | 89 |
| **H5** | SLIEEQLKTFLDKFLHELEDLFYQSSL-NH₂ | 38.39 | (1/1,0523) | 90 |
| **I5** | SLIEEQLKTFLDKFNHELEDLFYQSSL-NH₂ | 19,31 | (0/1,0347) | 91 |
| **H5/I5/N₃₃ /M₃₃** | SLIEEQLKTFLDKFMHELEDLFYQSSL-NH₂ | 31,42 | (0/1,0366) | 92 |
| **H6** | STLEEQLKTFLDKFLHELEDLFYQSSL-NH₂ | 36.11 | (1/1,0433) | 93 |
| **H7** | SLAEEQLKTFLDKFLHELEDLFYQSSL-NH₂ | 38.07 | (1/1,049) | 94 |
| **H8** | SALEEQLKTFLDKFLHELEDLFYQSSL-NH₂ | 43.52 | (1/1,049) checked | 95 |
| **I8** | SALEEQLKTFLDKFNHELEDLFYQSSL-NH₂ | 23,71 | (0/1,0315) | 96 |
| **H8/I8/N₃₃ /M₃₃** | SALEEQLKTFLDKFMHELEDLFYQSSL-NH₂ | 36,53 | (0/1,0333) | 97 |
| **H9** | SALEEQLKTFLDKFHELEDLFYQASL-NH₂ | 45,25 | (1/1,0510) | 98 |
| **I9** | SALEEQLKTFLDKFNHELEDLFYQASL-NH₂ | 24,03 | (0/1,0334) | 99 |
| **H9/I9/N₃₃ /M₃₃** | SALEEQLKTFLDKFMHELEDLFYQASL-NH₂ | 37,82 | (0/1,0353) | 100 |
| **H10** | SALEEQLKTFLDKFLHELEDLFYQSAL-NH₂ | 44,41 | (1/1,0510) | 101 |
| **I10** | SALEEQLKTFLDKFNHELEDLFYQSAL-NH₂ | 23,90 | (0/1,0334) | 102 |
| **H11** | SALEEQLKTFLDKFLHELEDLFYQAAL-NH₂ | 49,31 | (1/1,0529) | 103 |
| **I11** | SALEEQLKTFLDKFNHELEDLFYQAAL-NH₂ | 25,10 | (0/1,0353) | 104 |
| **H12** | SALAEQLKTFLDKFLHELEDLFYQASL-NH₂ | 29,65 | | 105 |
| **H13** | SALEEQLATFLDKFLHELEDLFYQASL-NH₂ | 24,06 | | 106 |
| **H14** | SALEEQLKTFLDKALHELEDLFYQASL-NH₂ | 42,13 | | 107 |
| **H15** | SALEEQLKTFLDKFLHELEDLAYQASL-NH₂ | 50,51 | (1/1,0500) | 108 |
| **I15** | SALEEQLKTFLDKFNHELEDLAYQASL-NH₂ | 25,40 | (0/1,0324) | 109 |
| **H15/I15/ N₃₃/M₃₃** | SALEEQLKTFLDKFMHELEDLAYQASL-NH₂ | 42,09 | (0/1,0343) | 110 |
| **H16** | SALEEQLKTFLDKFLHELEDLAYQSSL-NH₂ | 47,11 | | 111 |
| **H17** | SALEEQLKTFLDKFLHELEDLAYQLSL-NH₂ | 54,22 | | 112 |
| **H18** | SALEEQLKTFLDKFLHELEDLL YQASL-NH₂ | 55,19 | (1/1,0569) | 113 |
| **I18** | SALEEQLKTFLDKFNHELEDLLYQASL-NH₂ | 26,50 | (0/1,0393) | 114 |
| **H18/I18/ N₃₃/M₃₃** | SALEEQLKTFLDKFMHELEDLLYQASL-NH₂ | 46,25 | (0/1,0411) | 115 |
| **H19** | SLLEEQLKTFLDKFLHELEDLAYQASL-NH₂ | 47,42 | | 116 |
| **H20** | SALEEQLKTFLDKFLHELEDLLYQLSL-NH₂ | 63,71 | (1/1,0637) | 117 |
| **I20** | SALEEQLKTFLDKFNHELEDLLYQLSL-NH₂ | 29,62 | (0/1,0462) | 118 |
| **H20/I20/ N₃₃/M₃₃** | SALEEQLKTFLDKFMHELEDLLYQLSL-NH₂ | 54,20 | (0/1,0480) | 119 |
| **H21** | SALEEQLKTFLDKFLHELEDLLYQLAL-NH₂ | 77,64 | (1/1,0657) | 120 |
| **I21** | SALEEQLKTFLDKFNHELEDLLYQLAL-NH₂ | 38,32 | (0/1,0481) | 121 |
| **H21/I21/ N₃₃/M₃₃** | SALEEQLKTFLDKFMHELEDLL YQLAL-NH₂ | 68,18 | (0/1,0500) | 122 |
| **H22** | Ac-SALEEQLKTFLDKFLHELEDLLYQLAL-NH₂ | 66,48 | | 123 |
| **H23** | SAIEEQLKTFLDKFLHELEDLLYQLAL-NH₂ | 76,13 | (1/1,0620) | 124 |
| **I23** | SAIEEQLKTFLDKFNHELEDLLYQLAL-NH₂ | 35,91 | (0/1,0445) | 125 |
| **H23/I23/ N₃₃/M₃₃** | SAIEEQLKTFLDKFMHELEDLLYQLAL-NH₂ | 67,16 | (0/1,0463) | 126 |
| **H24** | SVLEEQLKTFLDKFLHELEDLLYQLAL-NH₂ | 69,60 | | 127 |
| **H25** | SILEEQLKTFLDKFLHELEDLLYQLAL-NH₂ | 70,91 | | 128 |
| **H26** | SALEEQLKTFLDKFLHELEPLLYQLAL-NH₂ | 32,06 | (1/1,0730) | 129 |
| **H27** | SALEEQLKTFLDKFLHELEDPLYQLAL-NH₂ | 33,95 | (1/1,0588) | 130 |
| **I27** | SALEEQLKTFLDKFNHELEDPLYQLAL-NH₂ | 23,28 | (0/1,0412) | 131 |
| **H27/I27/ N₃₃/M₃₃** | SALEEQLKTFLDKFMHELEDPLYQLAL-NH₂ | 30,20 | (0/1,0431) | 132 |
| **H28** | STIEEQAKTFLDKFNHEAEDLFYQSSLAS-NH₂ | 5,19 | (1/1,0104) | 133 |
| **H29** | SALEEQLKTFLDKFLHELEDLLYQLALAS-NH₂ | 79,35 | (1/1,0638) | 134 |
| **H29/I29/ N₃₃/M₃₃** | SALEEQLKTFLDKFMHELEDLL YQLALAS-NH₂ | 72,78 | (1/1,0549) | 135 |
| **H30** | SALEEQLKTFLDKFLHELEDLLYQLALAL-NH₂ | 87,84 | (1/1,0720) | 136 |
| **H30/I30/ N₃₃/M₃₃** | SALEEQLKTFLDKFMHELEDLL YQLALAL-NH₂ | 82,83 | (1/1,0573) | 137 |
| **I30** | SALEEQLKTFLDKFNHELEDLLYQLALAL-NH₂ | 54,08 | (1/1,0556) | 138 |
| **H31** | SALEEQYKTFLDKFLHELEDLLYQLALAL-NH₂ | 83,82 | (1/1,0689) | 139 |
| **H32** | SALEEQ(hY)KTFLDKFT HELFDLLYQLAL-NH₂ | 71,02 | (1/1,0624) | 140 |
| **H33** | SALEEQ(hY)KTFLDKFNHELEDLLYQLAL-NH₂ | 29,46 | (0/1,0448) | 141 |
| **H34** | SALEEQ(hY)KTFLDKFMHELEDLLYQLAL-NH₂ | 60,68 | (0/1,0467) | 142 |
| **H35** | SALEEQ(hY)KTFLDKFLHELEDPLYQLALAL -NH₂ | 26,21 | (1/1,0625) | 143 |
| **H36** | SALEEQ(hY)KTFLDKFNHELEDPLYQLAL-NH₂ | 18,70 | (0/1,0379) | 144 |
| **H37** | SALEEQ(hY)KTFLDKFMHELEDPLYQLAL-NH₂ | 23,66 | (0/1,0398) | 145 |
| **H38^{§}** | AHLFSYLTTKEEQDNDAIFLQEFSKES-NH₂ | 1,34 | (0/1,0083) | 148 |
| **H39^{§§}** | IEEQAKTFLDKFNHEAEDLFYQS-NH₂ | 1,09 | (0/1,0018) | 149 |

Noticeably, best helical contents were observed for peptide mimics with free N-terminus. Regarding the C-terminus, very similar results were observed for the C-carboxamide protected peptide mimics and the free carboxylate peptide. The C-terminus protected peptide was chosen for further development.

### 1. Optimization of the helical content

The first phase of the peptide mimics design was primarily focused on the optimization of the helical content. Considering that Asparagine (N) is known to disfavor helical folding, this amino acid was replaced by a Leucine (L) in order to increase the helicity (Peptide **H2**). The replacement of N by L led to a slight improvement of the helical content for at least a peptide with N-terminus free. This trend, *i.e.* free N-terminus improvement of helical content, is observed for all peptides calculated here. This property might be related to the capping box. Considering this observation, the C-amidated and N-free termini peptides were chosen when helical content discrepancies were not important between the totally free peptide and the N-and/or C-protected peptides. This choice is considered to be crucial, since protections of the N-and C- termini is known to increase stability toward proteolysis.

In a next step of the optimization process and in order to re-enforce the amphipathic character of the peptide hydrophobic interactions, a Leucine scan was realized on key positions A25 (Peptide **H3**) and A36 (Peptide **H4**). The A25 to L25 substitution led to a high increase of helical content, from 8,33 to 21,02% for the peptide with C-terminus protected (21,58% for N- and C-termini protected). The A36 to L36 substitution led again to a slight increase of helical content, from 21,02 to 32,54% for the C-terminus protected peptide.

The importance of the four residues in N-terminus was then evaluated considering the consensus sequence "SXXE" (SEQ ID NO: 147) reported as a capping box and considering that T₂₀ hydroxyl function is not involved in any interaction and that increasing hydrophobicity in this position might enhance the helical propensity. T₂₀ was first replaced by a Leucine (Peptide **H5**). The T₂₀/L₂₀ led again to an increase of the helical content with the same trends as previously described for N- and C- termini modifications, from 32,54% to 38,39%. A Leucine-scanning of the XX residues (*i.e.* TI) of this capping Box was then performed. If the same trends as previously described for N- and C- termini modifications were observed, the I₂₁/L₂₁ (Peptide **H6**) slightly improves the helical content as compared to peptide **H4.**

An Alanine-scanning was then realized on the capping box leading (Peptides **H7** and **H8**). In both cases, an improvement of the helical content was observed. The replacement of T₂₀ by A₂₀ increases the helical content compared to the parent peptide **H4** from 32,54% to 43,52% for peptide **H8** that was selected for further investigation.

Regarding both E residues of the capping box through the **Table 2,** indicating the high helical frequency of these residues, their mutation to A was not considered (a quick calculation was however performed with Agadir confirming this assumption).

Similarly, an Alanine-scanning was then performed on the two non-essential Serine residues of C-terminus sequence of peptide **H8** (43,89%). Peptides **H9** and **H10** were thus designed. The S₄₃/A₄₃ and S₄₄/A₄₄ mutations led to a light improvement compared to parent peptide **H8.** Two consecutive mutations of S43 and S44 were also investigated with peptide **H11.** The double S₄₃/A₄₃ and S₄₄/A₄₄ mutation led to an improvement as compared to parent peptide **H8,** from 43,52% to 49,31%. But since the aim is to design a peptide mimic very similar to the hACE2 α-helix, modification of one or two Serine residues in position 43 and 44 have to be considered to improve, for example, the physico-chemical properties of our mimics (Solubility, half-life...).

Several others mutations were also evaluated from peptide **H9** to peptide **H13** for which K₂₆ was mutated to A₂₆. A drastic drop in the helical content was here observed from 45,25% to 24,06%, highlighting a crucial role of this lysine. In comparison, the F₃₂/A₃₂ mutation of peptide **H9** to peptide **H14** did not led to any changes in the helical content, whereas the F₄₀/A₄₀ mutation in peptide **H15** from peptide **H9** led to a peptide with 50,51% of helical content. Similar mutation F₄₀/A₄₀ from peptide **H8** to **H16** led also to slight increase of the helical content, from 43,52% to 47,11%. Peptide **H15** was thus selected for further investigations starting from a Leucine-scan, since Leucine according to **Table 2** was known to increase peptide helicity. Indeed, the A₄₃/L₄₃ mutation from peptide **H15** to peptide **H17** led again to an improvement of the helical content, from 50,51% to 54,22%. Similar trends were observed for the A₄₀/L₄₀ mutation of peptide **H18,** from 50,51% to 55,19%. A new peptide was designed combining both mutations, A₄₀/L₄₀ and A₄₃/L₄₃ leading to peptide **H20**. A gap was reached here with 63,71% of helical content. Of note, the A₂₀/L₂₀ mutation from peptide **H15** to peptide **H19** led a slight decrease of the helical content, from 50,51% to 47,42%. Leucine is thus not recommended in this position.

Starting then from peptide **H20**, a new Alanine-scanning with S₄₃/A₄₃ mutation was performed. A clear jump was here observed, from 63,71% to 77,64% for peptide H21. Since increasing metabolic stability might be an important issue, its fully protected form was studied, from peptide **H21** to peptide **H22.** If a slight decrease was observed in the helical content from 77,64% to 66,48%, this decrease will only have a small impact on the energy binding of the system (If we refer to ΔG=-RTLnK, the energy gap shifts from 2.4KCal for a helical content from 5,51% to 77,64% and 1.5Kcal for a helical content shift from 10 to 50%).

A₂₀ of peptide **H21** was also mutated to V20 (Peptide **H24**) and I₂₀ (Peptide **H25**), leading only to a slight decrease of the helical content.

Finally, considering the kink, there were two possible choice to induce this camber of the α-helix. Indeed, the hydrogen-bond network is disrupted around two positions:
- 34O/38HN involving the oxygen of H₃₄ and the HN of D₃₈, and
- 35O/39HN involving the oxygen of E₃₅ and the HN of L₃₉.

This lack of hydrogen bond led us to consider the substitution of D₃₈ or L39 by Proline residues which in turn might help in kink inducing. Indeed, the cyclic constraint of proline is destabilizing helix because of the lack of hydrogen bonding but Proline is known as distortions inducer of transmembrane helix.

So, starting from peptide **H21,** D₃₈ was mutated to P₃₈ leading to peptide **H26.** Of note, D₃₈ is a crucial residue in the context of the native protein, involved in crucial interaction with protein S of SARCoV-2. Apart from structural impact, this mutation will lead to the loss of crucial interaction involving aspartate side chain. The introduction of prolino-aspartate might be helpful to recover the information brought by the side-chain. As expected, the Agadir calculation led to a drop in the helical content, from 77,64% to 32,06%. Nevertheless, this might lead to an increase in peptide binding thanks to limited entropy loss during interaction with protein S.

Since the L39 is not crucial in term of peptide/protein S interaction, its mutation form L39 to P39 seemed to be more suitable than the previous one. Indeed, again, apart from structural impact, this mutation will lead to the loss of Leucine side chain not involved in the direct interaction with the protein S of SARCoV-2. Nevertheless, useful cis and trans-3-substituted prolinoleucine analogues proven to be highly efficient constrained leucine surrogates and they might be used for affinity improvement if required. The peptide **H27** was thus drawn from peptide **H21** and its helical content calculated. As expected, a drop was again observed in the helical content, shifting from 77,64% to 33,95%, but this might lead to an increase in peptide binding thanks to limited entropy loss during interaction with protein S since the Leucine side chain is not involved in the interaction with SARCoV-2 protein S. Moreover, the Proline mutation might increase peptide mimic stability toward proteases.

Since the helical content might be crucial, 29mers peptide mimics were also investigated based on the native sequence in the context of hACE2. As expected, the helical content of the native peptide **H28** calculated with Agadir algorithm was very poor (5,19% for the C-carboxamide protected peptide, even less than the 27mers native sequence (peptide mimic **H1** bearing 5,51% of helical content) whereas the sequence modification according to mutation of peptide mimic **H21** led to the peptide mimic **H29** with 79,35% helical content. Finally, the mutation of S₄₇ to L₄₇ led then to peptide mimic **H30** with a helical content of 87,84% for the C-carboxamide protected analogue.

### 2. Identification of immunogenicity

The second phase of the peptide mimics maturation was related to the identification of immunogenicity. Indeed, since peptides triggering immune response might be neutralized and thus lose their therapeutic potential, the immunogenicity was predicted and optimized using the semi-empirical method reported by Kolaskar and Tongaonkar based on the physicochemical properties of amino acid residues and their frequencies of occurrence in experimentally known segmental epitopes (Kolaskar & Tongaonkar, FEBS Lett. 1990, 276(1-2):172-174). The data are reported in **Table 5** as the determination of the number of antigenic determinant (AD) and the average antigenic propensities (AAP) (http://imed.med.ucm.es/Tools/antigenic.pl). Using this approach, it was observed that if the replacement of N₃₃ to L₃₃ led to increasing helical content, it was also responsible of increasing calculated immunogenic propensities of our peptide mimics (**H1** to **H2**). Indeed, the mere mutation of N₃₃ (**H1**) to L₃₃ (**H2**) led to a shift from non-immunogenic peptide for **H1** to the presence of one antigenic determinant (AD) for peptide **H2.** In order to limit the immunogenicity, a screening of position 33 was performed with the 19 possible natural amino acids in order to identify which of them might limit the immunogenicity while retaining the helical content. Among them, only mutation by Methionine appeared to be crucial not only to avoid predicted immunogenicity but also for its ability in increasing helical content. This is exemplified by the study performed around peptide **H3** which appeared to present calculated immunogenicity. Indeed, peptide **I3** was designed from **H3.** If a decrease in the helical content was observed for **13,** shifting from 21.02% to 13.96%, the peptide **I3** appeared to be devoid of predicted immunogenicity. In an attempt to combine increasing helical content and lowering immunogenicity, we designed peptide **H3/I3** by mutation of N33 to A₃₃. If Alanine was known to increase helical content (shifting from 13.96% to 18,18%), this mutation led surprisingly to increasing immunogenicity. Moreover, two antigenic determinants appeared in the sequence framing the Alanine. It thus appeared that the N33 mutation is not suited and the peptide **I4** was thus designed from **H4** for which one antigenic calculated determinant appeared in the sequence. Peptide **14,** devoid of calculated immunogenicity, showed a very slight increase of helical content compared to peptide **I3** and the simple N₃₃/M₃₃ mutation led to recovery of the helical content without inducing immunogenicity.

In order to increase the helical content while retaining the absence of calculated immunogenicity, we designed peptide **I5** from peptide **H5** which led to an increase in helical content as compared to **I4** and did not led to calculated immunogenicity. **I8** was designed from **H8** and again, this peptide showed increased helical content as compared to **H5** and was devoid of calculated immunogenicity. **H9** showed calculated immunogenicity with one antigenic determinant and **I9** designed from **H9** did not show any calculated immunogenicity while being able to maintain helical content. Similar results were observed for **H11/I11, H15/I15, H18/I18, H20/I20, H21/I21** with each time a slight increase in helical content from **I11** to **I21** shifting from 25,10% to 38,32% of helical content. Finally, a screening with methionine was realized with most of the peptides devoid of immunogenicity (**H1, I3, I4, I8, I9, I15, I18, I20, I23)** and the best results combining high helical content (68,18%) together with absence of calculated immunogenicity was observed for the peptide derived from the combination of **H21/I21** with N33/M33 mutation (**H21/I21/N₃₃/M₃₃**). Attempt to increase the helical content by increasing the size of the peptide led to increasing calculated immunogenicity as exemplified with the **H29/I29/N₃₃/M₃₃** mutated peptide.

From the above analyses of helical content and immunogenicity, the following conclusions summing up the optimized required properties for the design of our peptide mimic were drawn:
i) a sequence of 29 residues can be modified for optimization of the helical content:
S₁₉T₂₀I₂₁**E₂₂**E₂₃Q₂₄A₂₅K₂₆T₂₇F₂₈L₂₉D₃₀K₃₁F₃₂N₃₃H₃₄E₃₅A₃₆E₃₇D₃₈L₃₉F₄₀Y₄₁Q₄₂S₄₃S₄₄L₄₅A₄₆S₄₇ (SEQ ID NO: 67)
Thirteen residues (highlighted) are crucial in the interaction of the sequence with the protein S of SARCov-2. Since a consensus sequence (SXXE, SEQ ID NO: 147) was identified as a capping box at the N-terminus side of the peptide and since capping box are described to enhance helicity, we proposed to classify E22 has a crucial residue (in bold and highlighted) for increasing the helical content in our optimization process while retaining the capping box
ii) Helical content versus immunogenicity:
The optimization process led to two peptides with high helical content, **H21** and **H30** (Table 6):

**Table 6. Two high helical content peptides.**

| Peptide name | Peptide sequence | Helical content % | Immunogenicity (AD/AAP) | SEQ ID NO |
|---|---|---|---|---|
| **H21** | SALEEQLKTFLDKFLHELEDLL YQLAL-NH₂ | 77,64 | (1/1,0657) | 120 |
| **H30** | SALEEQLKTFLDKFLHELEDLLYQLALAL-NH₂ | 87,84 | (1/1,0720) | 136 |

Adding two more residues to **H21** increased the helical content but was detrimental to the calculated immunogenicity. Indeed, if **H21** appeared to be immunogenic upon calculation, the mutation of L33 to N₃₃ led to a peptide mimic **121** devoid of calculated immunogenic properties but with lower helical content. The position 33 in the sequence appears to be the keystone of the calculated immunogenicity and recovery of a high helical content remains possible thanks to the mutation of the native N33 by M33 for 27mers peptide **H21/I21/N₃₃/M₃₃** (Table 7):

**Table 7. Position 33 is the keystone of calculated immunogenicity.**

| Peptide name | Peptide sequence | Helical content % | Immunogenicity (AD/AAP) | SEQ ID NO |
|---|---|---|---|---|
| **H21** | SALEEQLKTFLDKFLHELEDLLYQLAL-NH₂ | 77,64 | (1/1,0657) | 120 |
| **I21** | SALEEQLKTFLDKFNHFT EDLLYQLAL-NH₂ | 38,32 | (0/1,0481) | 121 |
| **H21/I21/ N₃₃/M₃₃** | SALEEQLKTFLDKFMHELEDLLYQLAL-NH₂ | 68,18 | (0/1,0500) | 122 |

In preliminary conclusion, the position 33 involves a residue (N33) not crucial for the interaction of the helix with the protein S but crucial for the helicity and for immunogenicity. The only possible mutation on this position is N33 to M33 to increase the helical content together with avoiding calculated immunogenicity.

Similar mutations were evaluated for the 29mers peptides with optimized helical content (Table 8):

**Table 8. Problems of immunogenicity remaining for the 29mers peptides.**

| Peptide name | Peptide sequence | Helical content % | Immunogenicity (AD/AAP) | SEQ ID NO |
|---|---|---|---|---|
| **H29** | SALEEQLKTFLDKFLHELEDLLYQLALAS-NH₂ | 79,35 | (1/1,0638) | 134 |
| **H29/I29/ N₃₃/M₃₃** | SALEEQLKTFLDKFMHELEDLLYQLALAS-NH₂ | 72,78 | (1/1,0549) | 135 |
| **H30** | SALEEQLKTFLDKFLHELEDLLYQLALAL-NH₂ | 87,84 | (1/1,0720) | 136 |
| **H30/I30/ N₃₃/M₃₃** | SALEEQLKTFLDKFMHELEDLLYQLALAL-NH₂ | 82,83 | (1/1,0573) | 137 |
| **130** | SALEEQLKTFLDKFNHELEDLLYQLALAL-NH₂ | 54,08 | (1/1,0556) | 138 |

In that case, whatever mutations evaluated (N33 or M33), all 29mers peptides remained with calculated immunogenicity leading us to conclude that increasing the size of our peptide in order to optimize the helical content is possible but may be detrimental to calculated immunogenicity.

### 3. Structural optimization around the kink

During the modeling study, a kink around D₃₈ and L₃₉ was identified and mutations D₃₈/P₃₈ and L₃₉/P₃₉ were considered (Table 9):

**Table 9. Mimicking the king with proline mutations.**

| Peptide name | Peptide sequence | Helical content % | Immunogenicity (AD/AAP) | SEQ ID NO |
|---|---|---|---|---|
| **H26** | SALEEQLKTFLDKFLHELEPLLYQLAL-NH₂ | 32,06 | (1/1,0730) | 129 |
| **H27** | SALEEQLKTFLDKFLHELEDPLYQLAL-NH₂ | 33,95 | (1/1,0588) | 130 |
| **I27** | SALEEQLKTFLDKFNHELEDPLYQLAL-NH₂ | 23,28 | (0/1,0412) | 131 |
| **H27/I27/ N₃₃/M₃₃** | SALEEQLKTFLDKFMHELEDPLYQLAL-NH₂ | 30,20 | (0/1,0431) | 132 |

If both D38/P38 and L39/P39 mutations proven by molecular modeling analyses to mimic the kink, D₃₈ is a crucial residue for the interaction with protein S of SARCoV-2 and thus the L39/P39 appeared suitable for further development. Indeed, if peptide H₂₇ appeared with a calculated immunogenicity, the mutation of L33 to N33 and M33 led to peptides **I27** and **H27/I27/ N33/M33** devoid of calculated immunogenicity.

### 4. Structural optimization around A25

The above molecular modeling studies highlighted out of the 29-mers α-helix, the proximity of Y₈₃ of hACE2 with F₂₈. Since this interaction might stabilize the overall structure of the complex, peptide mimics were designed to re-introduce the Y residue side chain instead of A25, a non-crucial residue ideally positioned for mutation. For that purpose, A25 was mutated to a Y₂₅ and homoY₂₅. MM calculation highlighted a better overlapping of aromatic ring with homoY instead of Y. Four peptides (**H31, H32, H33** and **H34**) were first designed and optimization of the Helical content/calculated immunogenicity ration led to peptide **H34** (Table 10).

**Table 10. Peptides designed to re-introduce Y83 side chain.**

| Peptide name | Peptide sequence | Helical content % | Immunogenicity (AD/AAP) *** | SEQ ID NO |
|---|---|---|---|---|
| **H31** | SALEEQYKTFLDKFLHELEDLLYQLALAL-NH₂ | 83,82 | (1/1,0689) | 139 |
| **H32** | SALEEQ (hY) KTFLDKFLHELEDLLYQLAL-NH₂ | 71,02 | (1/1,0624) | 140 |
| **H33** | SALEEQ (hY) KTFLDKFNHELEDLLYQLAL-NH₂ | 29,46 | (0/1,0448) | 141 |
| **H34** | SALEEQ (hY) KTFLDKFMHELEDLLYQLAL-NH₂ | 60,68 | (0/1,0467) | 142 |

### 5. Combination of the kink with Y₈₃ side chain

Three peptides were designed in order to combine the kink with Y₈₃ side chain (Table 11):

**Table 11. Peptide combining the kink and Y₈₃ side chain.**

| Peptide name* | Peptide sequence | Helical content % | Immunogenicity (AD/AAP) | SEQ ID NO |
|---|---|---|---|---|
| **H35** | SALEEQ (hY) KTFLDKFLHELEDPLYQLALAL-NH2 | 26,21 | (1/1,0625) | 143 |
| **H36** | SALEEQ (hY) KTFLDKFNHELEDPLYQLAL-NH₂ | 18,70 | (0/1,0379) | 144 |
| **H37** | SALEEQ (hY) KTFLDKFMHELEDPLYQLAL-NH₂ | 23,66 | (0/1,0398) | 145 |

Again, our will to optimize the helical content/calculated immunogenicity ratio led to peptide **H37.**

### 6. Peptides selected for structural and biological investigations

The following peptides were selected for further investigations of conformational propensities, binding properties and prophylactic effects (Table 12):

**Table 12. Peptides selected for further investigations.**

| Peptide name | Peptide sequence | SEQ ID NO |
|---|---|---|
| **H1** | STIEEQAKTFLDKFNHEAEDLFYQSSL-NH₂ | 80 |
| **H20** | SALEEQLKTFLDKFLHELEDLLYQLSL-NH₂ | 117 |
| **H21** | SALEEQLKTFLDKFLHELEDLLYQLAL-NH₂ | 120 |
| **H21/I21/ N₃₃/M₃₃** | SALEEQLKTFLDKFMHELEDLLYQLAL-NH₂ | 122 |
| **H23/I23/ N₃₃/M₃₃** | SAIEEQLKTFLDKFMHELEDLLYQLAL-NH₂ | 126 |
| **I27** | SALEEQLKTFLDKFNHELEDPLYQLAL-NH₂ | 131 |
| **H27/I27/ N₃₃/M₃₃** | SALEEQLKTFLDKFMHELEDPLYQLAL-NH₂ | 132 |
| **H30** | SALEEQLKTFLDKFLHELEDLLYQLALAL-NH₂ | 136 |
| **H32** | SALEEQ (hY) KTFLDKFLHELEDLLYQLAL-NH₂ | 140 |
| **H33** | SALEEQ (hY) KTFLDKFNHELEDLLYQLAL-NH₂ | 141 |
| **H34** | SALEEQ (hY) KTFLDKFMHELEDLLYQLAL-NH₂ | 142 |
| **H36** | SALEEQ (hY) KTFLDKFNHELEDPLYQLAL-NH₂ | 144 |
| **H37** | SALEEQ (hY) KTFLDKFMHELEDPLYQLAL-NH₂ | 145 |
| **H38** | AHLFSYLTTKEEQDNDAIFLQEFSKES-NH₂ | 148 |
| **H39** | IEEQAKTFLDKFNHEAEDLFYQS-NH₂ | 149 |

## Claims

1. A peptide comprising or consisting in an amino acid sequence of SEQ ID NO: 1:
S-X-X-X-X-Q-X-X-T-F-X-D-K-X-X-H-E-X-E-[D/P/mA]-X-X-Y-Q-X-X-L (SEQ ID NO: 1),
wherein X is any amino acid, and
mA is a N-methyl-alanine,
or a pharmaceutically acceptable salt thereof.

2. The peptide according to claim 1, wherein said peptide comprises or consists in an amino acid sequence of SEQ ID NO: 4:
S-T-I-E-X-Q-X-X-T-F-X-D-K-X-X-H-E-X-E-[D/P/mA]-X-X-Y-Q-X-X-L (SEQ ID NO: 4),
wherein X is any amino acid, and
mA is a N-methyl-alanine,
or a pharmaceutically acceptable salt thereof.

3. The peptide according to claim 1 or 2, wherein said peptide comprises or consists in an amino acid sequence of SEQ ID NO: 5: 
S-[T/L/A/V/I]-[I/L/A]-E-E-Q-[A/L/Y/hY]-[K/A]-T-F-L-D-K-[F/A]-[N/L/M]-H-E-[A/L/]-E-
[D/P/mA]-[L/P]-[F/A/L]-Y-Q-[S/L/A]-[S/A]-L (SEQ ID NO: 5),
wherein hY is an homotyrosine, and
mA is a N-methyl-alanine,
or a pharmaceutically acceptable salt thereof.

4. The peptide according to any one of claims 1 to 3, wherein said peptide comprises or consists in an amino acid sequence selected from the group consisting of: SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79 and SEQ ID NO: 80.

5. The peptide according to any one of claims 1 to 4, wherein said peptide is:
- a fully free peptide,
- a Nter-acetamido protected peptide,
- a Cter-carboxamide protected peptide, or
- a Nter-acetamido and Cter-carboxamide protected peptide.

6. The peptide as defined in any one of claims 1 to 5, for use as a medicament.

7. The peptide as defined in any one of claim 1 to 6, for use in the prevention and/or the treatment of a viral infection, preferably a coronavirus infection, more preferably an infection by a coronavirus selected from the list consisting of SARS-CoV, MERS-CoV and SARS-CoV2.

8. A pharmaceutical composition comprising at least (i) a peptide as defined in any one of claims 1 to 7, and (ii) a pharmaceutically acceptable carrier.

9. The pharmaceutical composition according to claim 8, wherein said pharmaceutical composition is in the form of a solution, a syrup, a tablet, a pill, an eye-wash, a powder, a spray, a capsule, a gum, a pastille, a lipstick or an emulsion.

10. A diagnostic or imaging reagent comprising a peptide according to any one of claims 1 to 7.

11. A polynucleotide encoding a peptide as defined in any one of claims 1 to 7.

12. An expression vector comprising a polynucleotide encoding a peptide as defined in claim 11.

13. A host cell modified with a polynucleotide as defined in claim 11 or an expression vector as defined in claim 12.

14. A pharmaceutical composition, comprising at least: (i) a polynucleotide encoding a peptide as defined in any one claims 1 to 7, and/or an expression vector comprising said polynucleotide, and (ii) a pharmaceutically acceptable carrier.


SEQUENCE LISTING

<110>  SORBONNE UNIVERSITE
       CENTRE NATIONAL DE LA RECHERCHE SCIENTIFIQUE
       ECOLE NORMALE SUPERIEURE

<120>  PEPTIDES FOR PREVENTING OR TREATING VIRAL INFECTIONS

<130>  F247800034/EP

<160>  149

<170>  PatentIn version 3.5

<210>  1
<211>  27
<212>  PRT
<213>  Artificial sequence

<220>
<223>  Synthetic peptide


<220>
<221>  MISC_FEATURE
<222>  (2)..(5)
<223>  Xaa = any amino acid

<220>
<221>  MISC_FEATURE
<222>  (7)..(8)
<223>  Xaa = any amino acid

<220>
<221>  MISC_FEATURE
<222>  (11)..(11)
<223>  Xaa = any amino acid

<220>
<221>  MISC_FEATURE
<222>  (14)..(15)
<223>  Xaa = any amino acid

<220>
<221>  MISC_FEATURE
<222>  (18)..(18)
<223>  Xaa = any amino acid

<220>
<221>  MISC_FEATURE
<222>  (20)..(20)
<223>  Xaa = D, P or N-methyl-Alanine

<220>
<221>  MISC_FEATURE
<222>  (21)..(22)
<223>  Xaa = any amino acid

<220>
<221>  MISC_FEATURE
<222>  (25)..(26)
<223>  Xaa = any amino acid

<400>  1

Ser Xaa Xaa Xaa Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu
            20                  25

<210>  2
<211>  27
<212>  PRT
<213>  Artificial sequence

<220>
<223>  Synthetic peptide

<220>
<221>  misc_feature
<222>  (2)..(3)
<223>  X = any amino acid

<220>
<221>  misc_feature
<222>  (5)..(5)
<223>  X = any amino acid

<220>
<221>  misc_feature
<222>  (7)..(8)
<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature
<222>  (25)..(26)
<223>  X = any amino acid

<400>  2

Ser Xaa Xaa Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu
            20                  25

<210>  3
<211>  27
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (5)..(5)
<223>  X = any amino acid

<220>
<221>  misc_feature
<222>  (7)..(8)
<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)

<223>  X = any amino acid

<220>
<221>  misc_feature
<222>  (25)..(26)
<223>  X = any amino acid

<400>  3

Ser Xaa Xaa Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu
            20                  25

<210>  4
<211>  27
<212>  PRT
<213>  Artificial sequence

<220>
<223>  Synthetic peptide

<220>
<221>  misc_feature
<222>  (5)..(5)
<223>  X = any amino acid

<220>
<221>  misc_feature
<222>  (7)..(8)
<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature

<222>  (25)..(26)
<223>  X = any amino acid

<400>  4

Ser Thr Ile Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu
            20                  25

<210>  5
<211>  27
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>
<221>  misc_feature
<222>  (14)..(14)
<223>  X = F or A

<220>
<221>  misc_feature
<222>  (15)..(15)
<223>  X = N, L or M

<220>
<221>  misc_feature
<222>  (18)..(18)
<223>  X = A or L

<220>
<221>  misc_feature
<222>  (20)..(20)
<223>  X = D, P or N-methyl-Alanine

<220>

<221>  misc_feature
<222>  (21)..(21)
<223>  X = L or P

<220>
<221>  misc_feature
<222>  (22)..(22)
<223>  X = F, A or L

<220>
<221>  misc_feature
<222>  (25)..(25)
<223>  X = S, L or A

<220>
<221>  misc_feature
<222>  (26)..(26)
<223>  X = S or A

<400>  5

Ser Xaa Xaa Glu Glu Gln Xaa Xaa Thr Phe Leu Asp Lys Xaa Xaa His
1               5                   10                  15

Glu Xaa Glu Xaa Xaa Xaa Tyr Gln Xaa Xaa Leu
            20                  25

<210>  6
<211>  27
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>
<221>  misc_feature
<222>  (14)..(14)
<223>  X = F or A

<220>
<221> misc_feature
<222> (18)..(18)
<223> X = A or L

<220>
<221> misc_feature
<222> (20)..(20)
<223> X = D, P or N-methyl-Alanine

<220>
<221> misc_feature
<222> (21)..(21)
<223> X = L or P

<220>
<221> misc_feature
<222> (22)..(22)
<223> X = F, A or L

<220>
<221> misc_feature
<222> (25)..(25)
<223> X = S, L or A

<220>
<221> misc_feature
<222> (26)..(26)
<223> X = S or A

<400> 6

Ser Xaa Xaa Glu Glu Gln Xaa Xaa Thr Phe Leu Asp Lys Xaa Met His
1               5                   10                  15

Glu Xaa Glu Xaa Xaa Xaa Tyr Gln Xaa Xaa Leu
            20                  25

<210> 7
<211> 29
<212> PRT
<213> Artificial sequence

<220>
<223> Synthetic peptide

<220>
<221> misc_feature
<222> (2)..(5)
<223> X = any amino acid

<220>
<221> misc_feature
<222> (7)..(8)
<223> X = any amino acid

<220>
<221> misc_feature
<222> (11)..(11)
<223> X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature
<222>  (25)..(26)
<223>  X = any amino acid

<220>
<221>  misc_feature
<222>  (28)..(29)
<223>  X = any amino acid

<400>  7

Ser Xaa Xaa Xaa Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu Xaa Xaa
            20                  25

<210>  8
<211>  29
<212>  PRT
<213>  Artificial sequence

<220>
<223>  Synthetic peptide

<220>
<221>  misc_feature
<222>  (2)..(3)
<223>  X = any amino acid

<220>
<221>  misc_feature
<222>  (5)..(5)
<223>  X = any amino acid

<220>
<221>  misc_feature
<222>  (7)..(8)

<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature
<222>  (25)..(26)
<223>  X = any amino acid

<220>
<221>  misc_feature
<222>  (28)..(29)
<223>  X = any amino acid

<400>  8

Ser Xaa Xaa Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu Xaa Xaa
            20                  25

<210>  9
<211>  29
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature

<222>  (3)..(3)
<223>  X = I, L or A

<220>
<221>  misc_feature
<222>  (5)..(5)
<223>  X = any amino acid

<220>
<221>  misc_feature
<222>  (7)..(8)
<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature
<222>  (25)..(26)
<223>  X = any amino acid

<220>
<221>  misc_feature
<222>  (28)..(29)
<223>  X = any amino acid

<400>  9

Ser Xaa Xaa Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu Xaa Xaa
            20                  25

<210>  10
<211>  29
<212>  PRT
<213>  Artificial sequence

<220>
<223>  Synthetic peptide

<220>
<221>  misc_feature
<222>  (5)..(5)
<223>  X = any amino acid

<220>
<221>  misc_feature
<222>  (7)..(8)
<223>  X = any amino acid

<220>
<221>  misc_feature
<222>  (11)..(11)
<223>  X = any amino acid

<220>
<221>  misc_feature
<222>  (14)..(15)
<223>  X = any amino acid

<220>
<221>  misc_feature
<222>  (18)..(18)
<223>  X = any amino acid

<220>
<221>  misc_feature
<222>  (20)..(20)
<223>  X = D, P or N-methyl-Alanine

<220>
<221>  misc_feature
<222>  (21)..(22)
<223>  X = any amino acid

<220>
<221>  misc_feature
<222>  (25)..(26)
<223>  X = any amino acid

<220>
<221>  misc_feature
<222>  (28)..(29)
<223>  X = any amino acid

<400>  10

Ser Thr Ile Glu Xaa Gln Xaa Xaa Thr Phe Xaa Asp Lys Xaa Xaa His
1               5                   10                  15

Glu Xaa Glu Xaa Xaa Xaa Tyr Gln Xaa Xaa Leu Xaa Xaa
            20                  25

<210>  11
<211>  29
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>
<221>  misc_feature
<222>  (14)..(14)
<223>  X = F or A

<220>
<221>  misc_feature
<222>  (15)..(15)
<223>  X = N, L or M

<220>
<221>  misc_feature
<222>  (18)..(18)
<223>  X = A or L

<220>
<221>  misc_feature
<222>  (20)..(20)
<223>  X = D, P or N-methyl-Alanine

<220>
<221>  misc_feature
<222>  (21)..(21)
<223>  X = L or P

<220>
<221>  misc_feature
<222>  (22)..(22)
<223>  X = F, A or L

<220>
<221>  misc_feature
<222>  (25)..(25)
<223>  X = S, L or A

<220>
<221>  misc_feature
<222>  (26)..(26)

<223>  X = S or A

<220>
<221>  misc_feature
<222>  (28)..(29)
<223>  X = any amino acid

<400>  11

Ser Xaa Xaa Glu Glu Gln Xaa Xaa Thr Phe Leu Asp Lys Xaa Xaa His
1               5                   10                  15

Glu Xaa Glu Xaa Xaa Xaa Tyr Gln Xaa Xaa Leu Xaa Xaa
            20                  25

<210>  12
<211>  29
<212>  PRT
<213>  Artificial sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>
<221>  misc_feature
<222>  (14)..(14)
<223>  X = F or A

<220>
<221>  misc_feature
<222>  (18)..(18)
<223>  X = A or L

<220>
<221>  misc_feature
<222>  (20)..(20)
<223>  X = D, P or N-methyl-Alanine

<220>
<221>  misc_feature

<222>  (21)..(21)
<223>  X = L or P

<220>
<221>  misc_feature
<222>  (22)..(22)
<223>  X = F, A or L

<220>
<221>  misc_feature
<222>  (25)..(25)
<223>  X = S, L or A

<220>
<221>  misc_feature
<222>  (26)..(26)
<223>  X = S or A

<220>
<221>  misc_feature
<222>  (28)..(29)
<223>  X = any amino acid

<400>  12

Ser Xaa Xaa Glu Glu Gln Xaa Xaa Thr Phe Leu Asp Lys Xaa Met His
1               5                   10                  15

Glu Xaa Glu Xaa Xaa Xaa Tyr Gln Xaa Xaa Leu Xaa Xaa
            20                  25

<210>  13
<211>  29
<212>  PRT
<213>  Artificial Sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>

<221>  misc_feature
<222>  (14)..(14)
<223>  X = F or A

<220>
<221>  misc_feature
<222>  (15)..(15)
<223>  X = N, L or M

<220>
<221>  misc_feature
<222>  (18)..(18)
<223>  X = A or L

<220>
<221>  misc_feature
<222>  (20)..(20)
<223>  X = D, P or N-methyl-Alanine

<220>
<221>  misc_feature
<222>  (21)..(21)
<223>  X = L or P

<220>
<221>  misc_feature
<222>  (22)..(22)
<223>  X = F, A or L

<220>
<221>  misc_feature
<222>  (25)..(25)
<223>  X = S, L or A

<220>
<221>  misc_feature
<222>  (26)..(26)
<223>  X = S or A

<220>
<221>  misc_feature
<222>  (29)..(29)
<223>  X = S or L

<400>  13

Ser Xaa Xaa Glu Glu Gln Xaa Xaa Thr Phe Leu Asp Lys Xaa Xaa His
1               5                   10                  15

Glu Xaa Glu Xaa Xaa Xaa Tyr Gln Xaa Xaa Leu Ala Xaa
            20                  25

<210>  14
<211>  29
<212>  PRT
<213>  Artificial Sequence

<220>
<223>  Synthetic peptide

<220>
<221>  misc_feature
<222>  (2)..(2)
<223>  X = T, L, A, V or I

<220>
<221>  misc_feature
<222>  (3)..(3)
<223>  X = I, L or A

<220>
<221>  misc_feature
<222>  (7)..(7)
<223>  X = A, L, Y or homotyrosine

<220>
<221>  misc_feature
<222>  (8)..(8)
<223>  X = K or A

<220>
<221>  misc_feature
<222>  (14)..(14)
<223>  X = F or A

<220>
<221>  misc_feature
<222>  (18)..(18)
<223>  X = A or L

<220>
<221>  misc_feature
<222>  (20)..(20)
<223>  X = D, P or N-methyl-Alanine

<220>
<221>  misc_feature
<222>  (21)..(21)
<223>  X = L or P

<220>
<221>  misc_feature
<222>  (22)..(22)
<223>  X = F, A or L

<220>
<221>  misc_feature
<222>  (25)..(25)
<223>  X = S, L or A

<220>
<221>  misc_feature
<222>  (26)..(26)
<223>  X = S or A

<220>
<221>  misc_feature
<222>  (29)..(29)
<223>  X = S or L

<400>  14

Ser Xaa Xaa Glu Glu Gln Xaa Xaa Thr Phe Leu Asp Lys Xaa Met His

1               5                   10                  15

Glu Xaa Glu Xaa Xaa Xaa Tyr Gln Xaa Xaa Leu Ala Xaa
            20                  25

<210>  15
<211>  27
<212>  PRT
<213>  Artificial Sequence

<220>
<223>  Synthetic peptide

<400>  15

Ser Thr Ile Glu Glu Gln Ala Lys Thr Phe Leu Asp Lys Phe Asn His
1               5                   10                  15

Glu Ala Glu Asp Leu Phe Tyr Gln Ser Ser Leu
            20                  25

<210>  16
<211>  27
<212>  PRT
<213>  Artificial Sequence

<220>
<223>  Synthetic peptide

<400>  16

Ser Thr Ile Glu Glu Gln Ala Lys Thr Phe Leu Asp Lys Phe Leu His
1               5                   10                  15

Glu Ala Glu Asp Leu Phe Tyr Gln Ser Ser Leu
            20                  25

<210>  17
<211>  27
<212>  PRT
<213>  Artificial Sequence

<220>
<223>  Synthetic peptide

<400>  17

Ser Thr Ile Glu Glu Gln Ala Lys Thr Phe Leu Asp Lys Phe Met His
1               5                   10                  15

Glu Ala Glu Asp Leu Phe Tyr Gln Ser Ser Leu
            20                  25

<210>  18
<211>  27

<212>  PRT
<213>  Artificial Sequence

<220>
<223>  Synthetic peptide

<400>  18

Ser Thr Ile Glu Glu Gln Leu Lys Thr Phe Leu Asp Lys Phe Leu His
1               5                   10                  15

Glu Ala Glu Asp Leu Phe Tyr Gln Ser Ser Leu
            20                  25

<210>  19
<211>  27
<212>  PRT
<213>  Artificial Sequence

<220>
<223>  Synthetic peptide

<400>  19

Ser Thr Ile Glu Glu Gln Leu Lys Thr Phe Leu Asp Lys Phe Asn His
1               5                   10                  15

Glu Ala Glu Asp Leu Phe Tyr Gln Ser Ser Leu
            20                  25

<210>  20
<211>  27
<212>  PRT
<213>  Artificial Sequence

<220>
<223>  Synthetic peptide

<400>  20

Ser Thr Ile Glu Glu Gln Leu Lys Thr Phe Leu Asp Lys Phe Met His
1               5                   10                  15

Glu Ala Glu Asp Leu Phe Tyr Gln Ser Ser Leu
            20                  25

<210>  21
<211>  27
<212>  PRT
<213>  Artificial Sequence

<220>
<223>  Synthetic peptide

<400>  21

Ser Thr Ile Glu Glu Gln Leu Lys Thr Phe Leu Asp Lys Phe Ala His

1               5                   10                  15

Glu Ala Glu Asp Leu Phe Tyr Gln Ser Ser Leu
            20                  25

<210>  22
<211>  27
<212>  PRT
<213>  Artificial Sequence

<220>
<223>  Synthetic peptide

<400>  22

Ser Thr Ile Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  23
<211>  27
<212>  PRT
<213>  Artificial Sequence

<220>
<223>  Synthetic peptide

<400>  23

Ser Thr Ile Glu Glu Gln Leu Lys Thr Phe Leu Asp Lys Phe Asn His
1               5                   10                  15

Glu Leu Glu Asp Leu Phe Tyr Gln Ser Ser Leu
            20                  25

<210>  24
<211>  27
<212>  PRT
<213>  Artificial Sequence

<220>
<223>  Synthetic peptide

<400>  24

Ser Thr Ile Glu Glu Gln Leu Lys Thr Phe Leu Asp Lys Phe Met His
1               5                   10                  15

Glu Leu Glu Asp Leu Phe Tyr Gln Ser Ser Leu
            20                  25

<210>  25
<211>  27

<212>  PRT
<213>  Artificial Sequence

<220>
<223>  Synthetic peptide

<400>  25

Ser Leu Ile Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  26
<211>  27
<212>  PRT
<213>  Artificial Sequence

<220>
<223>  Synthetic peptide

<400>  26

Ser Leu Ile Glu Glu Gln Leu Lys Thr Phe Leu Asp Lys Phe Asn His
1               5                   10                  15

Glu Leu Glu Asp Leu Phe Tyr Gln Ser Ser Leu
            20                  25

<210>  27
<211>  27
<212>  PRT
<213>  Artificial Sequence

<220>
<223>  Synthetic peptide

<400>  27

Ser Leu Ile Glu Glu Gln Leu Lys Thr Phe Leu Asp Lys Phe Met His
1               5                   10                  15

Glu Leu Glu Asp Leu Phe Tyr Gln Ser Ser Leu
            20                  25

<210>  28
<211>  27
<212>  PRT
<213>  Artificial Sequence

<220>
<223>  Synthetic peptide

<400>  28

Ser Thr Leu Glu Glu Gln Leu Lys Thr Phe Leu Asp Lys Phe Leu His

1               5                   10                  15

Glu Leu Glu Asp Leu Phe Tyr Gln Ser Ser Leu
            20                  25

<210>  29
<211>  27
<212>  PRT
<213>  Artificial Sequence

<220>
<223>  Synthetic peptide

<400>  29

Ser Leu Ala Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  30
<211>  27
<212>  PRT
<213>  Artificial Sequence

<220>
<223>  Synthetic peptide

<400>  30

Ser Ala Leu Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  31
<211>  27
<212>  PRT
<213>  Artificial Sequence

<220>
<223>  Synthetic peptide

<400>  31

Ser Ala Leu Glu Glu Gln Leu Lys Thr Phe Leu Asp Lys Phe Asn His
1               5                   10                  15

Glu Leu Glu Asp Leu Phe Tyr Gln Ser Ser Leu
            20                  25

<210>  32
<211>  27

<212>  PRT
<213>  Artificial Sequence

<220>
<223>  Synthetic peptide

<400>  32

Ser Ala Leu Glu Glu Gln Leu Lys Thr Phe Leu Asp Lys Phe Met His
1               5                   10                  15

Glu Leu Glu Asp Leu Phe Tyr Gln Ser Ser Leu
            20                  25

<210>  33
<211>  27
<212>  PRT
<213>  Artificial Sequence

<220>
<223>  Synthetic peptide

<400>  33

Ser Ala Leu Glu Glu Gln Leu Lys Thr Phe Leu Asp Lys Phe Leu His
1               5                   10                  15

Glu Leu Glu Asp Leu Phe Tyr Gln Ala Ser Leu
            20                  25

<210>  34
<211>  27
<212>  PRT
<213>  Artificial Sequence

<220>
<223>  Synthetic peptide

<400>  34

Ser Ala Leu Glu Glu Gln Leu Lys Thr Phe Leu Asp Lys Phe Asn His
1               5                   10                  15

Glu Leu Glu Asp Leu Phe Tyr Gln Ala Ser Leu
            20                  25

<210>  35
<211>  27
<212>  PRT
<213>  Artificial Sequence

<220>
<223>  Synthetic peptide

<400>  35

Ser Ala Leu Glu Glu Gln Leu Lys Thr Phe Leu Asp Lys Phe Met His

1               5                   10                  15

Glu Leu Glu Asp Leu Phe Tyr Gln Ala Ser Leu
            20                  25

<210>  36
<211>  27
<212>  PRT
<213>  Artificial Sequence

<220>
<223>  Synthetic peptide

<400>  36

Ser Ala Leu Glu Glu Gln Leu Lys Thr Phe Leu Asp Lys Phe Leu His
1               5                   10                  15

Glu Leu Glu Asp Leu Phe Tyr Gln Ser Ala Leu
            20                  25

<210>  37
<211>  27
<212>  PRT
<213>  Artificial Sequence

<220>
<223>  Synthetic peptide

<400>  37

Ser Ala Leu Glu Glu Gln Leu Lys Thr Phe Leu Asp Lys Phe Asn His
1               5                   10                  15

Glu Leu Glu Asp Leu Phe Tyr Gln Ser Ala Leu
            20                  25

<210>  38
<211>  27
<212>  PRT
<213>  Artificial Sequence

<220>
<223>  Synthetic peptide

<400>  38

Ser Ala Leu Glu Glu Gln Leu Lys Thr Phe Leu Asp Lys Phe Leu His
1               5                   10                  15

Glu Leu Glu Asp Leu Phe Tyr Gln Ala Ala Leu
            20                  25

<210>  39
<211>  27

<212>  PRT
<213>  Artificial Sequence

<220>
<223>  Synthetic peptide

<400>  39

Ser Ala Leu Glu Glu Gln Leu Lys Thr Phe Leu Asp Lys Phe Asn His
1               5                   10                  15

Glu Leu Glu Asp Leu Phe Tyr Gln Ala Ala Leu
            20                  25

<210>  40
<211>  27
<212>  PRT
<213>  Artificial Sequence

<220>
<223>  Synthetic peptide

<400>  40

Ser Ala Leu Ala Glu Gln Leu Lys Thr Phe Leu Asp Lys Phe Leu His
1               5                   10                  15

Glu Leu Glu Asp Leu Phe Tyr Gln Ala Ser Leu
            20                  25

<210>  41
<211>  27
<212>  PRT
<213>  Artificial Sequence

<220>
<223>  Synthetic peptide

<400>  41

Ser Ala Leu Glu Glu Gln Leu Ala Thr Phe Leu Asp Lys Phe Leu His
1               5                   10                  15

Glu Leu Glu Asp Leu Phe Tyr Gln Ala Ser Leu
            20                  25

<210>  42
<211>  27
<212>  PRT
<213>  Artificial Sequence

<220>
<223>  Synthetic peptide

<400>  42

Ser Ala Leu Glu Glu Gln Leu Lys Thr Phe Leu Asp Lys Ala Leu His

1               5                   10                  15

Glu Leu Glu Asp Leu Phe Tyr Gln Ala Ser Leu
            20                  25

<210>  43
<211>  27
<212>  PRT
<213>  Artificial Sequence

<220>
<223>  Synthetic peptide

<400>  43

Ser Ala Leu Glu Glu Gln Leu Lys Thr Phe Leu Asp Lys Phe Leu His
1               5                   10                  15

Glu Leu Glu Asp Leu Ala Tyr Gln Ala Ser Leu
            20                  25

<210>  44
<211>  27
<212>  PRT
<213>  Artificial Sequence

<220>
<223>  Synthetic peptide

<400>  44

Ser Ala Leu Glu Glu Gln Leu Lys Thr Phe Leu Asp Lys Phe Asn His
1               5                   10                  15

Glu Leu Glu Asp Leu Ala Tyr Gln Ala Ser Leu
            20                  25

<210>  45
<211>  27
<212>  PRT
<213>  Artificial Sequence

<220>
<223>  Synthetic peptide

<400>  45

Ser Ala Leu Glu Glu Gln Leu Lys Thr Phe Leu Asp Lys Phe Met His
1               5                   10                  15

Glu Leu Glu Asp Leu Ala Tyr Gln Ala Ser Leu
            20                  25

<210>  46
<211>  27

<212>  PRT
<213>  Artificial Sequence

<220>
<223>  Synthetic peptide

<400>  46

Ser Ala Leu Glu Glu Gln Leu Lys Thr Phe Leu Asp Lys Phe Leu His
1               5                   10                  15

Glu Leu Glu Asp Leu Ala Tyr Gln Ser Ser Leu
            20                  25

<210>  47
<211>  27
<212>  PRT
<213>  Artificial Sequence

<220>
<223>  Synthetic peptide

<400>  47

Ser Ala Leu Glu Glu Gln Leu Lys Thr Phe Leu Asp Lys Phe Leu His
1               5                   10                  15

Glu Leu Glu Asp Leu Ala Tyr Gln Leu Ser Leu
            20                  25

<210>  48
<211>  27
<212>  PRT
<213>  Artificial Sequence

<220>
<223>  Synthetic peptide

<400>  48

Ser Ala Leu Glu Glu Gln Leu Lys Thr Phe Leu Asp Lys Phe Leu His
1               5                   10                  15

Glu Leu Glu Asp Leu Leu Tyr Gln Ala Ser Leu
            20                  25

<210>  49
<211>  27
<212>  PRT
<213>  Artificial Sequence

<220>
<223>  Synthetic peptide

<400>  49

Ser Ala Leu Glu Glu Gln Leu Lys Thr Phe Leu Asp Lys Phe Asn His

1               5                   10                  15


Glu Leu Glu Asp Leu Leu Tyr Gln Ala Ser Leu
            20                  25



<210>  50
<211>  27
<212>  PRT
<213>  Artificial Sequence

<220>
<223>  Synthetic peptide

<400>  50

Ser Ala Leu Glu Glu Gln Leu Lys Thr Phe Leu Asp Lys Phe Met His
1               5                   10                  15


Glu Leu Glu Asp Leu Leu Tyr Gln Ala Ser Leu
            20                  25



<210>  51
<211>  27
<212>  PRT
<213>  Artificial Sequence

<220>
<223>  Synthetic peptide

<400>  51

Ser Leu Leu Glu Glu Gln Leu Lys Thr Phe Leu Asp Lys Phe Leu His
1               5                   10                  15


Glu Leu Glu Asp Leu Ala Tyr Gln Ala Ser Leu
            20                  25



<210>  52
<211>  27
<212>  PRT
<213>  Artificial Sequence

<220>
<223>  Synthetic peptide

<400>  52

Ser Ala Leu Glu Glu Gln Leu Lys Thr Phe Leu Asp Lys Phe Leu His
1               5                   10                  15


Glu Leu Glu Asp Leu Leu Tyr Gln Leu Ser Leu
            20                  25



<210>  53
<211>  27

<212>  PRT
<213>  Artificial Sequence

<220>
<223>  Synthetic peptide

<400>  53

Ser Ala Leu Glu Glu Gln Leu Lys Thr Phe Leu Asp Lys Phe Asn His
1               5                   10                  15

Glu Leu Glu Asp Leu Leu Tyr Gln Leu Ser Leu
            20                  25

<210>  54
<211>  27
<212>  PRT
<213>  Artificial Sequence

<220>
<223>  Synthetic peptide

<400>  54

Ser Ala Leu Glu Glu Gln Leu Lys Thr Phe Leu Asp Lys Phe Met His
1               5                   10                  15

Glu Leu Glu Asp Leu Leu Tyr Gln Leu Ser Leu
            20                  25

<210>  55
<211>  27
<212>  PRT
<213>  Artificial Sequence

<220>
<223>  Synthetic peptide

<400>  55

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  56
<211>  27
<212>  PRT
<213>  Artificial Sequence

<220>
<223>  Synthetic peptide

<400>  56

Ser Ala Leu Glu Glu Gln Leu Lys Thr Phe Leu Asp Lys Phe Asn His

1               5                   10                  15

Glu Leu Glu Asp Leu Leu Tyr Gln Leu Ala Leu
            20                  25

<210>  57
<211>  27
<212>  PRT
<213>  Artificial Sequence

<220>
<223>  Synthetic peptide

<400>  57

Ser Ala Leu Glu Glu Gln Leu Lys Thr Phe Leu Asp Lys Phe Met His
1               5                   10                  15

Glu Leu Glu Asp Leu Leu Tyr Gln Leu Ala Leu
            20                  25

<210>  58
<211>  27
<212>  PRT
<213>  Artificial Sequence

<220>
<223>  Synthetic peptide

<400>  58

Ser Ala Ile Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  59
<211>  27
<212>  PRT
<213>  Artificial Sequence

<220>
<223>  Synthetic peptide

<400>  59

Ser Ala Ile Glu Glu Gln Leu Lys Thr Phe Leu Asp Lys Phe Asn His
1               5                   10                  15

Glu Leu Glu Asp Leu Leu Tyr Gln Leu Ala Leu
            20                  25

<210>  60
<211>  27

<212>  PRT
<213>  Artificial Sequence

<220>
<223>  Synthetic peptide

<400>  60

Ser Ala Ile Glu Glu Gln Leu Lys Thr Phe Leu Asp Lys Phe Met His
1               5                   10                  15

Glu Leu Glu Asp Leu Leu Tyr Gln Leu Ala Leu
            20                  25

<210>  61
<211>  27
<212>  PRT
<213>  Artificial Sequence

<220>
<223>  Synthetic peptide

<400>  61

Ser Val Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  62
<211>  27
<212>  PRT
<213>  Artificial Sequence

<220>
<223>  Synthetic peptide

<400>  62

Ser Ile Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  63
<211>  27
<212>  PRT
<213>  Artificial Sequence

<220>
<223>  Synthetic peptide

<400>  63

Ser Ala Leu Glu Glu Gln Leu Lys Thr Phe Leu Asp Lys Phe Leu His

1               5                   10                  15


Glu Leu Glu Pro Leu Leu Tyr Gln Leu Ala Leu
            20                  25



<210>  64
<211>  27
<212>  PRT
<213>  Artificial Sequence

<220>
<223>  Synthetic peptide

<400>  64

Ser Ala Leu Glu Glu Gln Leu Lys Thr Phe Leu Asp Lys Phe Leu His
1               5                   10                  15


Glu Leu Glu Asp Pro Leu Tyr Gln Leu Ala Leu
            20                  25



<210>  65
<211>  27
<212>  PRT
<213>  Artificial Sequence

<220>
<223>  Synthetic peptide

<400>  65

Ser Ala Leu Glu Glu Gln Leu Lys Thr Phe Leu Asp Lys Phe Asn His
1               5                   10                  15


Glu Leu Glu Asp Pro Leu Tyr Gln Leu Ala Leu
            20                  25



<210>  66
<211>  27
<212>  PRT
<213>  Artificial Sequence

<220>
<223>  Synthetic peptide

<400>  66

Ser Ala Leu Glu Glu Gln Leu Lys Thr Phe Leu Asp Lys Phe Met His
1               5                   10                  15


Glu Leu Glu Asp Pro Leu Tyr Gln Leu Ala Leu
            20                  25



<210>  67
<211>  29

<212>  PRT
<213>  Artificial Sequence

<220>
<223>  Synthetic peptide

<400>  67

Ser Thr Ile Glu Glu Gln Ala Lys Thr Phe Leu Asp Lys Phe Asn His
1               5                   10                  15

Glu Ala Glu Asp Leu Phe Tyr Gln Ser Ser Leu Ala Ser
            20                  25

<210>  68
<211>  29
<212>  PRT
<213>  Artificial Sequence

<220>
<223>  Synthetic peptide

<400>  68

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu Ala Ser
            20                  25

<210>  69
<211>  29
<212>  PRT
<213>  Artificial Sequence

<220>
<223>  Synthetic peptide

<400>  69

Ser Ala Leu Glu Glu Gln Leu Lys Thr Phe Leu Asp Lys Phe Met His
1               5                   10                  15

Glu Leu Glu Asp Leu Leu Tyr Gln Leu Ala Leu Ala Ser
            20                  25

<210>  70
<211>  29
<212>  PRT
<213>  Artificial Sequence

<220>
<223>  Synthetic peptide

<400>  70

Ser Ala Leu Glu Glu Gln Leu Lys Thr Phe Leu Asp Lys Phe Leu His

1               5                   10                  15

Glu Leu Glu Asp Leu Leu Tyr Gln Leu Ala Leu Ala Leu
            20                  25

<210>  71
<211>  29
<212>  PRT
<213>  Artificial Sequence

<220>
<223>  Synthetic peptide

<400>  71

Ser Ala Leu Glu Glu Gln Leu Lys Thr Phe Leu Asp Lys Phe Met His
1               5                   10                  15

Glu Leu Glu Asp Leu Leu Tyr Gln Leu Ala Leu Ala Leu
            20                  25

<210>  72
<211>  29
<212>  PRT
<213>  Artificial Sequence

<220>
<223>  Synthetic peptide

<400>  72

Ser Ala Leu Glu Glu Gln Leu Lys Thr Phe Leu Asp Lys Phe Asn His
1               5                   10                  15

Glu Leu Glu Asp Leu Leu Tyr Gln Leu Ala Leu Ala Leu
            20                  25

<210>  73
<211>  29
<212>  PRT
<213>  Artificial Sequence

<220>
<223>  Synthetic peptide

<400>  73

Ser Ala Leu Glu Glu Gln Tyr Lys Thr Phe Leu Asp Lys Phe Leu His
1               5                   10                  15

Glu Leu Glu Asp Leu Leu Tyr Gln Leu Ala Leu Ala Leu
            20                  25

<210>  74
<211>  27

<212>  PRT
<213>  Artificial Sequence

<220>
<223>  Synthetic peptide


<220>
<221>  misc_feature
<222>  (7)..(7)
<223>  X = homotyrosine

<400>  74

Ser Ala Leu Glu Glu Gln Xaa Lys Thr Phe Leu Asp Lys Phe Leu His
1               5                   10                  15


Glu Leu Glu Asp Leu Leu Tyr Gln Leu Ala Leu
            20                  25


<210>  75
<211>  27
<212>  PRT
<213>  Artificial Sequence

<220>
<223>  Synthetic peptide


<220>
<221>  misc_feature
<222>  (7)..(7)
<223>  X = homotyrosine

<400>  75

Ser Ala Leu Glu Glu Gln Xaa Lys Thr Phe Leu Asp Lys Phe Asn His
1               5                   10                  15


Glu Leu Glu Asp Leu Leu Tyr Gln Leu Ala Leu
            20                  25


<210>  76
<211>  27
<212>  PRT
<213>  Artificial Sequence

<220>
<223>  Synthetic peptide


<220>
<221>  misc_feature
<222>  (7)..(7)
<223>  X = homotyrosine

<400>  76

Ser Ala Leu Glu Glu Gln Xaa Lys Thr Phe Leu Asp Lys Phe Met His

1               5                   10                  15

Glu Leu Glu Asp Leu Leu Tyr Gln Leu Ala Leu
            20                  25

<210>  77
<211>  29
<212>  PRT
<213>  Artificial Sequence

<220>
<223>  Synthetic peptide

<220>
<221>  misc_feature
<222>  (7)..(7)
<223>  X = homotyrosine

<400>  77

Ser Ala Leu Glu Glu Gln Xaa Lys Thr Phe Leu Asp Lys Phe Leu His
1               5                   10                  15

Glu Leu Glu Asp Pro Leu Tyr Gln Leu Ala Leu Ala Leu
            20                  25

<210>  78
<211>  27
<212>  PRT
<213>  Artificial Sequence

<220>
<223>  Synthetic peptide

<220>
<221>  misc_feature
<222>  (7)..(7)
<223>  X = homotyrosine

<400>  78

Ser Ala Leu Glu Glu Gln Xaa Lys Thr Phe Leu Asp Lys Phe Asn His
1               5                   10                  15

Glu Leu Glu Asp Pro Leu Tyr Gln Leu Ala Leu
            20                  25

<210>  79
<211>  27
<212>  PRT
<213>  Artificial Sequence

<220>
<223>  Synthetic peptide

<220>
<221>  misc_feature
<222>  (7)..(7)
<223>  X = homotyrosine

<400>  79

Ser Ala Leu Glu Glu Gln Xaa Lys Thr Phe Leu Asp Lys Phe Met His
1               5                   10                  15

Glu Leu Glu Asp Pro Leu Tyr Gln Leu Ala Leu
            20                  25

<210>  80
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  80

Ser Thr Ile Glu Glu Gln Ala Lys Thr Phe Leu Asp Lys Phe Asn His
1               5                   10                  15

Glu Ala Glu Asp Leu Phe Tyr Gln Ser Ser Leu
            20                  25

<210>  81
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  81

Ser Thr Ile Glu Glu Gln Ala Lys Thr Phe Leu Asp Lys Phe Leu His
1               5                   10                  15

Glu Ala Glu Asp Leu Phe Tyr Gln Ser Ser Leu
            20                  25

<210>  82
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  82

Ser Thr Ile Glu Glu Gln Ala Lys Thr Phe Leu Asp Lys Phe Met His
1               5                   10                  15


Glu Ala Glu Asp Leu Phe Tyr Gln Ser Ser Leu
            20                  25


<210>  83
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  83

Ser Thr Ile Glu Glu Gln Leu Lys Thr Phe Leu Asp Lys Phe Leu His
1               5                   10                  15


Glu Ala Glu Asp Leu Phe Tyr Gln Ser Ser Leu
            20                  25


<210>  84
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  84

Ser Thr Ile Glu Glu Gln Leu Lys Thr Phe Leu Asp Lys Phe Asn His
1               5                   10                  15

Glu Ala Glu Asp Leu Phe Tyr Gln Ser Ser Leu
            20                  25

<210>  85
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  85

Ser Thr Ile Glu Glu Gln Leu Lys Thr Phe Leu Asp Lys Phe Met His
1               5                   10                  15

Glu Ala Glu Asp Leu Phe Tyr Gln Ser Ser Leu
            20                  25

<210>  86
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  86

Ser Thr Ile Glu Glu Gln Leu Lys Thr Phe Leu Asp Lys Phe Ala His
1               5                   10                  15

Glu Ala Glu Asp Leu Phe Tyr Gln Ser Ser Leu
            20                  25

<210>  87
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  87

Ser Thr Ile Glu Glu Gln Leu Lys Thr Phe Leu Asp Lys Phe Leu His
1               5                   10                  15


Glu Leu Glu Asp Leu Phe Tyr Gln Ser Ser Leu
            20                  25


<210>  88
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  88

Ser Thr Ile Glu Glu Gln Leu Lys Thr Phe Leu Asp Lys Phe Asn His
1               5                   10                  15


Glu Leu Glu Asp Leu Phe Tyr Gln Ser Ser Leu
            20                  25


<210>  89
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  89

Ser Thr Ile Glu Glu Gln Leu Lys Thr Phe Leu Asp Lys Phe Met His
1               5                   10                  15

Glu Leu Glu Asp Leu Phe Tyr Gln Ser Ser Leu
            20                  25

<210>  90
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  90

Ser Leu Ile Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  91
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  91

Ser Leu Ile Glu Glu Gln Leu Lys Thr Phe Leu Asp Lys Phe Asn His
1               5                   10                  15

Glu Leu Glu Asp Leu Phe Tyr Gln Ser Ser Leu
            20                  25

<210>  92
<211>  27
<212>  PRT
<213>  Artificial Sequence

<220>
<223>  Synthetic peptide

<220>
<221>  misc_feature

<222>  (27)..(27)
<223>  C-ter = carboxamide

<400>  92

Ser Leu Ile Glu Glu Gln Leu Lys Thr Phe Leu Asp Lys Phe Met His
1               5                   10                  15

Glu Leu Glu Asp Leu Phe Tyr Gln Ser Ser Leu
            20                  25

<210>  93
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  93

Ser Thr Leu Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  94
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  94

Ser Leu Ala Glu Glu Gln Leu Lys Thr Phe Leu Asp Lys Phe Leu His
1               5                   10                  15

Glu Leu Glu Asp Leu Phe Tyr Gln Ser Ser Leu
            20                  25

<210>  95
<211>  27

<212>  PRT
<213>  Artificial Sequence

<220>
<223>  Synthetic peptide


<220>
<221>  misc_feature
<222>  (27)..(27)
<223>  C-ter = carboxamide

<400>  95

Ser Ala Leu Glu Glu Gln Leu Lys Thr Phe Leu Asp Lys Phe Leu His
1               5                   10                  15


Glu Leu Glu Asp Leu Phe Tyr Gln Ser Ser Leu
            20                  25


<210>  96
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  96

Ser Ala Leu Glu Glu Gln Leu Lys Thr Phe Leu Asp Lys Phe Asn His
1               5                   10                  15


Glu Leu Glu Asp Leu Phe Tyr Gln Ser Ser Leu
            20                  25


<210>  97
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  97

Ser Ala Leu Glu Glu Gln Leu Lys Thr Phe Leu Asp Lys Phe Met His

1               5                   10                  15

Glu Leu Glu Asp Leu Phe Tyr Gln Ser Ser Leu
            20                  25

<210>  98
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  98

Ser Ala Leu Glu Glu Gln Leu Lys Thr Phe Leu Asp Lys Phe Leu His
1               5                   10                  15

Glu Leu Glu Asp Leu Phe Tyr Gln Ala Ser Leu
            20                  25

<210>  99
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  99

Ser Ala Leu Glu Glu Gln Leu Lys Thr Phe Leu Asp Lys Phe Asn His
1               5                   10                  15

Glu Leu Glu Asp Leu Phe Tyr Gln Ala Ser Leu
            20                  25

<210>  100
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  100

Ser Ala Leu Glu Glu Gln Leu Lys Thr Phe Leu Asp Lys Phe Met His
1               5                   10                  15

Glu Leu Glu Asp Leu Phe Tyr Gln Ala Ser Leu
            20                  25

<210>  101
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  101

Ser Ala Leu Glu Glu Gln Leu Lys Thr Phe Leu Asp Lys Phe Leu His
1               5                   10                  15

Glu Leu Glu Asp Leu Phe Tyr Gln Ser Ala Leu
            20                  25

<210>  102
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  102

Ser Ala Leu Glu Glu Gln Leu Lys Thr Phe Leu Asp Lys Phe Asn His
1               5                   10                  15

Glu Leu Glu Asp Leu Phe Tyr Gln Ser Ala Leu
            20                  25

<210>  103
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  103

Ser Ala Leu Glu Glu Gln Leu Lys Thr Phe Leu Asp Lys Phe Leu His
1               5                   10                  15


Glu Leu Glu Asp Leu Phe Tyr Gln Ala Ala Leu
            20                  25


<210>  104
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  104

Ser Ala Leu Glu Glu Gln Leu Lys Thr Phe Leu Asp Lys Phe Asn His
1               5                   10                  15


Glu Leu Glu Asp Leu Phe Tyr Gln Ala Ala Leu
            20                  25


<210>  105
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  105

Ser Ala Leu Ala Glu Gln Leu Lys Thr Phe Leu Asp Lys Phe Leu His
1               5                   10                  15

Glu Leu Glu Asp Leu Phe Tyr Gln Ala Ser Leu
            20                  25

<210>  106
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  106

Ser Ala Leu Glu Glu Gln Leu Ala Thr Phe Leu Asp Lys Phe Leu His
1               5                   10                  15

Glu Leu Glu Asp Leu Phe Tyr Gln Ala Ser Leu
            20                  25

<210>  107
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  107

Ser Ala Leu Glu Glu Gln Leu Lys Thr Phe Leu Asp Lys Ala Leu His
1               5                   10                  15

Glu Leu Glu Asp Leu Phe Tyr Gln Ala Ser Leu
            20                  25

<210>  108
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  108

Ser Ala Leu Glu Glu Gln Leu Lys Thr Phe Leu Asp Lys Phe Leu His
1               5                   10                  15


Glu Leu Glu Asp Leu Ala Tyr Gln Ala Ser Leu
            20                  25


<210>  109
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  109

Ser Ala Leu Glu Glu Gln Leu Lys Thr Phe Leu Asp Lys Phe Asn His
1               5                   10                  15


Glu Leu Glu Asp Leu Ala Tyr Gln Ala Ser Leu
            20                  25


<210>  110
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  110

Ser Ala Leu Glu Glu Gln Leu Lys Thr Phe Leu Asp Lys Phe Met His
1               5                   10                  15

Glu Leu Glu Asp Leu Ala Tyr Gln Ala Ser Leu
            20                  25

<210>  111
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  111

Ser Ala Leu Glu Glu Gln Leu Lys Thr Phe Leu Asp Lys Phe Leu His
1               5                   10                  15

Glu Leu Glu Asp Leu Ala Tyr Gln Ser Ser Leu
            20                  25

<210>  112
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  112

Ser Ala Leu Glu Glu Gln Leu Lys Thr Phe Leu Asp Lys Phe Leu His
1               5                   10                  15

Glu Leu Glu Asp Leu Ala Tyr Gln Leu Ser Leu
            20                  25

<210>  113
<211>  27
<212>  PRT
<213>  Artificial Sequence

<220>
<223>  Synthetic peptide

<220>
<221>  misc_feature

<222>  (27)..(27)
<223>  C-ter = carboxamide

<400>  113

Ser Ala Leu Glu Glu Gln Leu Lys Thr Phe Leu Asp Lys Phe Leu His
1               5                   10                  15

Glu Leu Glu Asp Leu Leu Tyr Gln Ala Ser Leu
            20                  25

<210>  114
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  114

Ser Ala Leu Glu Glu Gln Leu Lys Thr Phe Leu Asp Lys Phe Asn His
1               5                   10                  15

Glu Leu Glu Asp Leu Leu Tyr Gln Ala Ser Leu
            20                  25

<210>  115
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  115

Ser Ala Leu Glu Glu Gln Leu Lys Thr Phe Leu Asp Lys Phe Met His
1               5                   10                  15

Glu Leu Glu Asp Leu Leu Tyr Gln Ala Ser Leu
            20                  25

<210>  116
<211>  27

<212>  PRT
<213>  Artificial Sequence

<220>
<223>  Synthetic peptide


<220>
<221>  misc_feature
<222>  (27)..(27)
<223>  C-ter = carboxamide

<400>  116

Ser Leu Leu Glu Glu Gln Leu Lys Thr Phe Leu Asp Lys Phe Leu His
1               5                   10                  15


Glu Leu Glu Asp Leu Ala Tyr Gln Ala Ser Leu
            20                  25


<210>  117
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  117

Ser Ala Leu Glu Glu Gln Leu Lys Thr Phe Leu Asp Lys Phe Leu His
1               5                   10                  15


Glu Leu Glu Asp Leu Leu Tyr Gln Leu Ser Leu
            20                  25


<210>  118
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  118

Ser Ala Leu Glu Glu Gln Leu Lys Thr Phe Leu Asp Lys Phe Asn His

1               5                   10                  15

Glu Leu Glu Asp Leu Leu Tyr Gln Leu Ser Leu
            20                  25

<210>  119
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  119

Ser Ala Leu Glu Glu Gln Leu Lys Thr Phe Leu Asp Lys Phe Met His
1               5                   10                  15

Glu Leu Glu Asp Leu Leu Tyr Gln Leu Ser Leu
            20                  25

<210>  120
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  120

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  121
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  121

Ser Ala Leu Glu Glu Gln Leu Lys Thr Phe Leu Asp Lys Phe Asn His
1               5                   10                  15


Glu Leu Glu Asp Leu Leu Tyr Gln Leu Ala Leu
            20                  25


<210>  122
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  122

Ser Ala Leu Glu Glu Gln Leu Lys Thr Phe Leu Asp Lys Phe Met His
1               5                   10                  15


Glu Leu Glu Asp Leu Leu Tyr Gln Leu Ala Leu
            20                  25


<210>  123
<211>  27
<212>  PRT
<213>  Artificial Sequence

<220>
<223>  Synthetic peptide


<220>
<221>  misc_feature
<222>  (1)..(1)
<223>  N-ter = acetamide

<220>
<221>  misc_feature
<222>  (27)..(27)
<223>  C-ter = carboxamide

<400>  123

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  124
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  124

Ser Ala Ile Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  125
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  125

Ser Ala Ile Glu Glu Gln Leu Lys Thr Phe Leu Asp Lys Phe Asn His
1               5                   10                  15

Glu Leu Glu Asp Leu Leu Tyr Gln Leu Ala Leu
            20                  25

<210>  126
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  126

Ser Ala Ile Glu Glu Gln Leu Lys Thr Phe Leu Asp Lys Phe Met His
1               5                   10                  15

Glu Leu Glu Asp Leu Leu Tyr Gln Leu Ala Leu
            20                  25

<210>  127
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  127

Ser Val Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  128
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  128

Ser Ile Leu Glu Glu Gln Leu Lys Thr Phe Leu Asp Lys Phe Leu His
1               5                   10                  15

Glu Leu Glu Asp Leu Leu Tyr Gln Leu Ala Leu
            20                  25

<210>  129
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  129

Ser Ala Leu Glu Glu Gln Leu Lys Thr Phe Leu Asp Lys Phe Leu His
1               5                   10                  15


Glu Leu Glu Pro Leu Leu Tyr Gln Leu Ala Leu
            20                  25


<210>  130
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  130

Ser Ala Leu Glu Glu Gln Leu Lys Thr Phe Leu Asp Lys Phe Leu His
1               5                   10                  15


Glu Leu Glu Asp Pro Leu Tyr Gln Leu Ala Leu
            20                  25


<210>  131
<211>  27
<212>  PRT
<213>  Artificial Sequence

<220>
<223>  Synthetic peptide


<220>
<221>  misc_feature
<222>  (27)..(27)
<223>  C-ter = carboxamide

<400>  131

Ser Ala Leu Glu Glu Gln Leu Lys Thr Phe Leu Asp Lys Phe Asn His
1               5                   10                  15

Glu Leu Glu Asp Pro Leu Tyr Gln Leu Ala Leu
            20                  25

<210>  132
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  132

Ser Ala Leu Glu Glu Gln Leu Lys Thr Phe Leu Asp Lys Phe Met His
1               5                   10                  15

Glu Leu Glu Asp Pro Leu Tyr Gln Leu Ala Leu
            20                  25

<210>  133
<211>  29
<212>  PRT
<213>  Artificial Sequence

<220>
<223>  Synthetic peptide

<220>
<221>  misc_feature
<222>  (29)..(29)
<223>  C-ter = carboxamide

<400>  133

Ser Thr Ile Glu Glu Gln Ala Lys Thr Phe Leu Asp Lys Phe Asn His
1               5                   10                  15

Glu Ala Glu Asp Leu Phe Tyr Gln Ser Ser Leu Ala Ser
            20                  25

<210>  134
<211>  29
<212>  PRT
<213>  Artificial Sequence

<220>

<223>  Synthetic peptide

<220>
<221>  misc_feature
<222>  (29)..(29)
<223>  C-ter = carboxamide

<400>  134

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu Ala Ser
            20                  25

<210>  135
<211>  29
<212>  PRT
<213>  Artificial Sequence

<220>
<223>  Synthetic peptide

<220>
<221>  misc_feature
<222>  (29)..(29)
<223>  C-ter = carboxamide

<400>  135

Ser Ala Leu Glu Glu Gln Leu Lys Thr Phe Leu Asp Lys Phe Met His
1               5                   10                  15

Glu Leu Glu Asp Leu Leu Tyr Gln Leu Ala Leu Ala Ser
            20                  25

<210>  136
<211>  29
<212>  PRT
<213>  Artificial Sequence

<220>
<223>  Synthetic peptide

<220>
<221>  misc_feature
<222>  (29)..(29)
<223>  C-ter = carboxamide

<400>  136

Ser Ala Leu Glu Glu Gln Leu Lys Thr Phe Leu Asp Lys Phe Leu His
1               5                   10                  15

Glu Leu Glu Asp Leu Leu Tyr Gln Leu Ala Leu Ala Leu

20                  25

<210>  137
<211>  29
<212>  PRT
<213>  Artificial Sequence

<220>
<223>  Synthetic peptide


<220>
<221>  misc_feature
<222>  (29)..(29)
<223>  C-ter = carboxamide

<400>  137

Ser Ala Leu Glu Glu Gln Leu Lys Thr Phe Leu Asp Lys Phe Met His
1               5                   10                  15


Glu Leu Glu Asp Leu Leu Tyr Gln Leu Ala Leu Ala Leu
            20                  25


<210>  138
<211>  29
<212>  PRT
<213>  Artificial Sequence

<220>
<223>  Synthetic peptide


<220>
<221>  misc_feature
<222>  (29)..(29)
<223>  C-ter = carboxamide

<400>  138

Ser Ala Leu Glu Glu Gln Leu Lys Thr Phe Leu Asp Lys Phe Asn His
1               5                   10                  15


Glu Leu Glu Asp Leu Leu Tyr Gln Leu Ala Leu Ala Leu
            20                  25


<210>  139
<211>  29
<212>  PRT
<213>  Artificial Sequence

<220>
<223>  Synthetic peptide


<220>
<221>  misc_feature
<222>  (29)..(29)

<223>  C-ter = carboxamide

<400>  139

Ser Ala Leu Glu Glu Gln Tyr Lys Thr Phe Leu Asp Lys Phe Leu His
1               5                   10                  15

Glu Leu Glu Asp Leu Leu Tyr Gln Leu Ala Leu Ala Leu
            20                  25

<210>  140
<211>  27
<212>  PRT
<213>  Artificial Sequence

<220>
<223>  Synthetic peptide

<220>
<221>  misc_feature
<222>  (7)..(7)
<223>  X = homotyrosine

<220>
<221>  misc_feature
<222>  (27)..(27)
<223>  C-ter = carboxamide

<400>  140

Ser Ala Leu Glu Glu Gln Xaa Lys Thr Phe Leu Asp Lys Phe Leu His
1               5                   10                  15

Glu Leu Glu Asp Leu Leu Tyr Gln Leu Ala Leu
            20                  25

<210>  141
<211>  27
<212>  PRT
<213>  Artificial Sequence

<220>
<223>  Synthetic peptide

<220>
<221>  misc_feature
<222>  (7)..(7)
<223>  X = homotyrosine

<220>
<221>  misc_feature
<222>  (27)..(27)
<223>  C-ter = carboxamide

<400>  141

Ser Ala Leu Glu Glu Gln Xaa Lys Thr Phe Leu Asp Lys Phe Asn His

1               5                   10                  15

Glu Leu Glu Asp Leu Leu Tyr Gln Leu Ala Leu
            20                  25

<210>  142
<211>  27
<212>  PRT
<213>  Artificial Sequence

<220>
<223>  Synthetic peptide

<220>
<221>  misc_feature
<222>  (7)..(7)
<223>  X = homotyrosine

<220>
<221>  misc_feature
<222>  (27)..(27)
<223>  C-ter = carboxamide

<400>  142

Ser Ala Leu Glu Glu Gln Xaa Lys Thr Phe Leu Asp Lys Phe Met His
1               5                   10                  15

Glu Leu Glu Asp Leu Leu Tyr Gln Leu Ala Leu
            20                  25

<210>  143
<211>  29
<212>  PRT
<213>  Artificial Sequence

<220>
<223>  Synthetic peptide

<220>
<221>  misc_feature
<222>  (7)..(7)
<223>  X = homotyrosine

<220>
<221>  misc_feature
<222>  (29)..(29)
<223>  C-ter = carboxamide

<400>  143

Ser Ala Leu Glu Glu Gln Xaa Lys Thr Phe Leu Asp Lys Phe Leu His
1               5                   10                  15

Glu Leu Glu Asp Pro Leu Tyr Gln Leu Ala Leu Ala Leu
            20                  25

<210>  144
<211>  27
<212>  PRT
<213>  Artificial Sequence

<220>
<223>  Synthetic peptide


<220>
<221>  misc_feature
<222>  (7)..(7)
<223>  X = homotyrosine

<220>
<221>  misc_feature
<222>  (27)..(27)
<223>  C-ter = carboxamide

<400>  144

Ser Ala Leu Glu Glu Gln Xaa Lys Thr Phe Leu Asp Lys Phe Asn His
1               5                   10                  15


Glu Leu Glu Asp Pro Leu Tyr Gln Leu Ala Leu
            20                  25


<210>  145
<211>  27
<212>  PRT
<213>  Artificial Sequence

<220>
<223>  Synthetic peptide


<220>
<221>  misc_feature
<222>  (7)..(7)
<223>  X = homotyrosine

<220>
<221>  misc_feature
<222>  (27)..(27)
<223>  C-ter = carboxamide

<400>  145

Ser Ala Leu Glu Glu Gln Xaa Lys Thr Phe Leu Asp Lys Phe Met His
1               5                   10                  15


Glu Leu Glu Asp Pro Leu Tyr Gln Leu Ala Leu
            20                  25


<210>  146
<211>  18
<212>  PRT

<213>  Artificial Sequence

<220>
<223>  Synthetic peptide

<400>  146

Glu Ala Glu Lys Ala Ala Lys Glu Ala Glu Lys Ala Ala Lys Glu Ala
1               5                   10                  15

Glu Lys

<210>  147
<211>  4
<212>  PRT
<213>  Artificial Sequence

<220>
<223>  Capping box pattern

<220>
<221>  misc_feature
<222>  (2)..(3)
<223>  X = any amino acid

<400>  147

Ser Xaa Xaa Glu
1

<210>  148
<211>  27
<212>  PRT
<213>  Artificial Sequence

<220>
<223>  Synthetic peptide

<220>
<221>  misc_feature
<222>  (27)..(27)
<223>  C-ter = carboxamide

<400>  148

Ala His Leu Phe Ser Tyr Leu Thr Thr Lys Glu Glu Gln Asp Asn Asp
1               5                   10                  15

Ala Ile Phe Leu Gln Glu Phe Ser Lys Glu Ser
            20                  25

<210>  149
<211>  23
<212>  PRT
<213>  Artificial Sequence

<220>
<223>  Synthetic peptide

<220>
<221>  misc_feature
<222>  (23)..(23)
<223>  C-ter = carboxamide

<400>  149

Ile Glu Glu Gln Ala Lys Thr Phe Leu Asp Lys Phe Asn His Glu Ala
1               5                   10                  15

Glu Asp Leu Phe Tyr Gln Ser
            20